# EUROPEAN PATENT SPECIFICATION

(11) **EP 1 326 896 B1**
(45) Date of publication and mention of the grant of the patent: **24.11.2010**
(21) Application number: 01979395.9
(22) Date of filing: 02.10.2001
(51) Int. Cl.: C07K 16/28, C12N 15/13, A61K 39/395, A61P 37/00

(54) **HUMAN ANTI-CD40 ANTIBODIES**
HUMANE ANTIKÖRPER GEGEN CD40
ANTICORPS HUMAINS DIRIGES CONTRE CD40

(30) Priority: 02.10.2000 US 237556 P
(43) Date of publication of application: 16.07.2003
(73) Proprietor: Novartis Vaccines and Diagnostics, Inc., Emeryville, CA 94608 (US)
(72) Inventor: CHU, Keting, Burlingame, CA 94010 (US); WANG, Changyu, El Cerrito, CA 94530 (US); YOSHIHARA, Corrine, Emeryville, CA 94608 (US); DONNELLY, John, J., Emeryville, CA 94608 (US)
(74) Representative: Marshall, Cameron John
(86) International application number: PCT/US2001/030857
(87) International publication number: WO 2002/028904

(56) References cited:
- EP-A- 0 945 465
- WO-A-01/83755
- WO-A-96/34096
- P. KARLSSON ET AL.: "Selection of human single chain antibodies against CD40." IMMUNOLOGY LETTERS, vol. 73, no. 2-3, September 2000 (2000-09), page 161 XP001073504 Amsterdam, The Netherlands

### SEQUENCE LISTING

<110> Chu, Keting
   Wang, Changyu
   Yoshihara, Corrine
   Donnelly, John J.
<120> HUMAN ANTI-CD40 ANTIBODIES
<130> 200130.527P1/16092.001
<140> US
   <141> 2000-10-02
<160> 18
<170> FastSEQ for Windows Version 4.0
<210> 1
   <211> 978
   <212> DNA
   <213> Homo sapiens
<400> 1
   gcctccacca agggcccatc ggtcttcccc ctggcgccct gctccaggag cacctccgag     60
   agcacagcgg ccctgggctg cctggtcaag gactacttcc ccgaaccggt gacggtgtcg    120
   tggaactcag gcgctctgac cagcggcgtg cacaccttcc cagctgtcct acagtcctca    180
   ggactctact ccctcagcag cgtggtgacc gtgccctcca gcaacttcgg cacccagacc    240
   tacacctgca acgtagatca caagcccagc aacaccaagg tggacaagac agttgagcgc    300
   aaatgttgtg tcgagtgccc accgtgccca gcaccacctg tggcaggacc gtcagtcttc    360
   ctcttccccc caaaacccaa ggacaccctc atgatctccc ggacccctga ggtcacgtgc    420
   gtggtggtgg acgtgagcca cgaagacccc gaggtccagt tcaactggta cgtggacggc    480
   gtggaggtgc ataatgccaa gacaaagcca cgggaggagc agttcaacag cacgttccgt    540
   gtggtcagcg tcctcaccgt tgtgcaccag gactggctga acggcaagga gtacaagtgc    600
   aaggtctcca acaaaggcct cccagccccc atcgagaaaa ccatctccaa aaccaaaggg    660
   cagccccgag aaccacaggt gtacaccctg cccccatccc gggaggagat gaccaagaac    720
   caggtcagcc tgacctgcct ggtcaaaggc ttctacccca gcgacatcgc cgtggagtgg    780
   gagagcaatg ggcagccgga gaacaactac aagaccacac ctcccatgct ggactccgac    840
   ggctccttct tcctctacag caagctcacc gtggacaaga gcaggtggca gcaggggaac    900
   gtcttctcat gctccgtgat gcatgaggct ctgcacaacc actacacgca gaagagcctc    960
   tccctgtctc cgggtaaa                                                  978
<210> 2
   <211> 326
   <212> PRT
   <213> Homo sapiens
<400> 2
   Ala Ser Thr Lys Gly Pro Ser Val Phe Pro Leu Ala Pro Cys Ser Arg
   1               5                   10                  15
   Ser Thr Ser Glu Ser Thr Ala Ala Leu Gly Cys Leu Val Lys Asp Tyr
                   20                  25                  30
   Phe Pro Glu Pro Val Thr Val Ser Trp Asn Ser Gly Ala Leu Thr Ser
               35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Asn Phe Gly Thr Gln Thr
   65                  70                  75                  80
   Tyr Thr Cys Asn Val Asp His Lys Pro Ser Asn Thr Lys Val Asp Lys
                   85                  90                  95
   Thr Val Glu Arg Lys Cys Cys Val Glu Cys Pro Pro Cys Pro Ala Pro
               100                 105                 110
   Pro Val Ala Gly Pro Ser Val Phe Leu Phe Pro Pro Lys Pro Lys Asp
           115                 120                 125
   Thr Leu Met Ile Ser Arg Thr Pro Glu Val Thr Cys Val Val Val Asp
       130                 135                 140
   Val Ser His Glu Asp Pro Glu Val Gln Phe Asn Trp Tyr Val Asp Gly
   145                 150                 155                 160
   Val Glu Val His Asn Ala Lys Thr Lys Pro Arg Glu Glu Gln Phe Asn
                   165                 170                 175
   Ser Thr Phe Arg Val Val Ser Val Leu Thr Val Val His Gln Asp Trp
               180                 185                 190
   Leu Asn Gly Lys Glu Tyr Lys Cys Lys Val Ser Asn Lys Gly Leu Pro
           195                 200                 205
   Ala Pro Ile Glu Lys Thr Ile Ser Lys Thr Lys Gly Gln Pro Arg Glu
       210                 215                 220
   Pro Gln Val Tyr Thr Leu Pro Pro Ser Arg Glu Glu Met Thr Lys Asn
   225                 230                 235                 240
   Gln Val Ser Leu Thr Cys Leu Val Lys Gly Phe Tyr Pro Ser Asp Ile
                   245                 250                 255
   Ala Val Glu Trp Glu Ser Asn Gly Gln Pro Glu Asn Asn Tyr Lys Thr
               260                 265                 270
   Thr Pro Pro Met Leu Asp Ser Asp Gly Ser Phe Phe Leu Tyr Ser Lys
           275                 280                 285
   Leu Thr Val Asp Lys Ser Arg Trp Gln Gln Gly Asn Val Phe Ser Cys
       290                 295                 300
   Ser Val Met His Glu Ala Leu His Asn His Tyr Thr Gln Lys Ser Leu
   305                 310                 315                 320
   Ser Leu Ser Pro Gly Lys
                   325
<210> 3
   <211> 324
   <212> DNA
   <213> Homo sapiens
<400> 3
   gaaattgttt tgacgcagtc tccaggcacc ctgtctttgt ctccagggga aggagccacc     60
   ctctcctgca gggccagtca gagtgttagc tacagctact tagcctggta ccagcagaaa    120
   cctggccagg ctcccaggct cctcatctat ggtgcatcca gcagggccac tggcatccca    180
   gacaggttca gtggcagtgg gtctggaaca gacttcactc tcaccatcag cagactggag    240
   cctgaagatt ttgcagtgta ttactgtcat cagtatggta actcatttcg gacgttcggc    300
   caagggacca aggtggaaat caaa                                           324
<210> 4
   <211> 348
   <212> DNA
   <213> Homo sapiens
<400> 4
   caggtgctgc aggagtctgg gggaggcgtg gtccagcctg ggaggtccct gagactctcc     60
   tgtgcagcct ctggattcac cttcagtagc tatggcatgg actgggtccg ccaggctcca    120
   ggcaaggggc tggagtgggt ggcagtttta tcatatgctg gaagtaataa atactatgca    180
   gactcagtga agggccgatt caccatctcc agagacaatt ccaagaacac gctgtatctg    240
   caaatgaaca gcctgagacc tgaggacacg gctgtgtatt actgtgcgcg agatacagtg    300
   aggggctttg actactgggg ccagggaatc ctggtcaccg tctcctca                 348
<210> 5
   <211> 336
   <212> DNA
   <213> Homo sapiens
<400> 5
   gatgttttga tgacccaaac tccactctcc ctgcctgtca gtcttggaga tcaagcctcc     60
   atctcttgca gatctagtca gaccattgta catagtaatg gacacaccta tttagaatgg    120
   tacctgcaga aaccaggcca gtctccaaag ctcctgatct ccaaagtttc caaccgattt    180
   tctggggtcc cagacaggtt cagtggcagt ggatcaggga cagatttcac actcaagatc    240
   agcagagtgg agcctgagga tctgggaatt tattattgct ttcaaggttc acatgttcca    300
   ttcactttcg gccctgggac caaagtgtat atcaaa                             336
<210> 6
   <211> 354
   <212> DNA
   <213> Homo sapiens
<400> 6
   caggtgcagc tgcaggagtc tgggggaggc gtggtccagc ctgggaggtc cctgagactc     60
   tcctgtgcag cctctggatt caccttcagt agctatggca tgcactgggt ccgccaggct    120
   ccaggcaagg ggctggagtg ggtggcagtt atatcatatg atggaagtat taaatactat    180
   gctgactccg tgaagggccg attcaccatc tccagagaca attccaagaa cacgctgtat    240
   ctgcaaatga acagcctgag agctgaggac acggctgtgt actactgtgc gagagatcat    300
   gggagtaacc cccttgacta ctggggccag ggaaccctgg tcaccgtctc ctca          354
<210> 7
   <211> 336
   <212> DNA
   <213> Homo sapiens
<400> 7
   gatattgtga tgacccagtc tccactctct ctgtccgtcg cccctggaca gccggcctcc     60
   atctcctgta agtctagtca gagcctcctg gagagttatg gagagaccta tttgtattgg    120
   tacctgcaga agccaggcca gcctccacag ctcctgatct atgcagtttt taagcggttc    180
   tctggagtgc cagataggtt cagtggcagc gggtcaggga cagatttcac actgaaaatc    240
   agccgggtgg aggctgagga tgttggggtt tattactgca tgcaaagtat gcagcttcct    300
   ctcactttcg gcggagggac caaggtggag atcaaa                             336
<210> 8
   <211> 366
   <212> DNA
   <213> Homo sapiens
<400> 8
   caggtgcagc tgcaggagtc tgggggaggc gtggtccagc ctgggaggtc cctgagactc     60
   tcctgtgcag cctctggatt caccttcaat aactttggca tacactgggt ccgccaggct    120
   ccaggcaagg ggctggagtg ggtggcagtt atatcatatg atggaagtga taaatattat    180
   gcagactccg tgaagggccg attcaccatc tccagagaca attccaagaa cacgctgaat    240
   ctgcaaatga atagtctgag agctgaggac acggctgtgt attactgtgc gagagatcgt    300
   cggtattact accactacta cggtatggac gtctggggcc aagggaccat ggtcaccgtc    360
   tcctca                                                                366
<210> 9
   <211> 527
   <212> DNA
   <213> Homo sapiens
<400> 9
   tggacatata tatgggtgac aatgacatcc actttgcctt tctctccaca ggcgcgcact     60
   cccaggtgca gctgcagcag tctggggctg aggtgaagaa gcctggggcc tcagtgaagg    120
   tctcctgcaa ggcttctgga tacaccttca ccgactacta tatgcactgg gtgcgacagg    180
   cccctggaca aggacttgag tggatgggat ggatcaaccc taacagtggt ggcacaaact    240
   atgcacagaa gtttcagggc agggtcacca tgaccaggga cacgtccatc agtatagcct    300
   acatggagct ggacaggctg agatctgacg acacggccgt gtattactgt gcgagagatg    360
   agatcctagc agcagatggt atctactatt tctacggtct ggacgtctgg ggccaaggga    420
   ccacggtcac cgtctcctca ggtgagtcct gtcgacggat ccacccaatg cccatgagcc    480
   cagacactgg acgctgaacc tcgcggacag ttaagaaccc aggggcc                   527
<210> 10
   <211> 351
   <212> DNA
   <213> Homo sapiens
<400> 10
   caggtgcagc tgcaggagtc tgggggaggc gtggtccagc ctgggaggtc cctgagactc     60
   tcctgtgcag cctctggatt caccttcagt agctatggca tggactgggt ccgccaggct    120
   ccaggcaagg ggctggagtg ggtggcagtt ttatcatatg atggaagtaa taagtactat    180
   gcagactcag tgaagggccg attcaccatc tccagagaca attccaagaa cacactgtat    240
   ctgcaattga acagcctgag agctgaggac acggctgtgt attactgtgc gcgagataca    300
   gtgaggggct ttgactactg gggccaggga atcctggtca ccgtctcctc a              351
<210> 11
   <211> 118
   <212> PRT
   <213> Homo sapiens
<400> 11
   Gln Val Gln Leu Gln Glu Ser Gly Gly Gly Val Val Gln Pro Gly Arg
    1               5                  10                  15
   Ser Leu Arg Leu Ser Cys Ala Ala Ser Gly Phe Thr Phe Ser Ser Tyr
               20                  25                  30
   Gly Met His Trp Val Arg Gln Ala Pro Gly Lys Gly Leu Glu Trp Val
           35                  40                  45
   Ala Val Ile Ser Tyr Asp Gly Ser Ile Lys Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp His Gly Ser Asn Pro Leu Asp Tyr Trp Gly Gln Gly Thr
               100                 105                 110
   Leu Val Thr Val Ser Ser
           115
<210> 12
   <211> 117
   <212> PRT
   <213> Homo sapiens
<400> 12
   Gln Val Gln Leu Gln Glu Ser Gly Gly Gly Val Val Gln Pro Gly Arg
    1               5                  10                  15
   Ser Leu Arg Leu Ser Cys Ala Ala Ser Gly Phe Thr Phe Ser Ser Tyr
               20                  25                  30
   Gly Met Asp Trp Val Arg Gln Ala Pro Gly Lys Gly Leu Glu Trp Val
           35                  40                  45
   Ala Val Leu Ser Tyr Asp Gly Ser Asn Lys Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Leu Asn Ser Leu Arg Ala Glu Asp Thr Ala Val Tyr Tyr Cys
                   85                  90                  95
   Ala Arg Asp Thr Val Arg Gly Phe Asp Tyr Trp Gly Gln Gly Ile Leu
               100                 105                 110
   Val Thr Val Ser Ser
           115
<210> 13
   <211> 122
   <212> PRT
   <213> Homo sapiens
<400> 13
   Gln Val Gln Leu Gln Glu Ser Gly Gly Gly Val Val Gln Pro Gly Arg
    1               5                  10                  15
   Ser Leu Arg Leu Ser Cys Ala Ala Ser Gly Phe Thr Phe Asn Asn Phe
               20                  25                  30
   Gly Ile His Trp Val Arg Gln Ala Pro Gly Lys Gly Leu Glu Trp Val
           35                  40                  45
   Ala Val Ile Ser Tyr Asp Gly Ser Asp Lys Tyr Tyr Ala Asp Ser Val
       50                  55                  60
   Lys Gly Arg Phe Thr Ile Ser Arg Asp Asn Ser Lys Asn Thr Leu Asn
   65                  70                  75                  80
   Leu Gln Met Asn Ser Leu Arg Ala Glu Asp Thr Ala Val Tyr Tyr Cys
                   85                  90                  95
   Ala Arg Asp Arg Arg Tyr Tyr Tyr His Tyr Tyr Gly Met Asp Val Trp
               100                 105                 110
   Gly Gln Gly Thr Met Val Thr Val Ser Ser
           115                 120
<210> 14
   <211> 117
   <212> PRT
   <213> Homo sapiens
<400> 14
   Gln Val Gln Leu Gln Glu Ser Gly Gly Gly Val Val Gln Pro Gly Arg
    1               5                  10                  15
   Ser Leu Arg Leu Ser Cys Ala Ala Ser Gly Phe Thr Phe Ser Ser Tyr
               20                  25                  30
   Gly Met Asp Trp Val Arg Gln Ala Pro Gly Lys Gly Leu Glu Trp Val
           35                  40                  45
   Ala Val Leu Ser Tyr Ala Gly Ser Asn Lys Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Pro Glu Asp Thr Ala Val Tyr Tyr Cys
                   85                  90                  95
   Ala Arg Asp Thr Val Arg Gly Phe Asp Tyr Trp Gly Gln Gly Ile Leu
               100                 105                 110
   Val Thr Val Ser Ser
           115
<210> 15
   <211> 112
   <212> PRT
   <213> Homo sapiens
<400> 15
   Asp Val Leu Met Thr Gln Thr Pro Leu Ser Leu Pro Val Ser Leu Gly
   1 5 10 15
   Asp Gln Ala Ser Ile Ser Cys Arg Ser Ser Gln Thr Ile Val His Ser
   20 25 30
   Asn Gly His Thr Tyr Leu Glu Trp Tyr Leu Gln Lys Pro Gly Gln Ser
   35 40 45
   Pro Lys Leu Leu Ile Ser Lys Val Ser Asn Arg Phe Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Pro Glu Asp Leu Gly Ile Tyr Tyr Cys Phe Gln Gly
   85 90 95
   Ser His Val Pro Phe Thr Phe Gly Pro Gly Thr Lys Val Tyr Ile Lys
   100 105 110
<210> 16
   <211> 105
   <212> PRT
   <213> Homo sapiens
<400> 16
   Glu Ile Val Leu Thr Gln Ser Pro Gly Thr Leu Ser Leu Ser Pro Gly
   1 5 10 15
   Glu Arg Ala Thr Leu Ser Cys Arg Ala Ser Gln Ser Val Ser Tyr Ser
   20 25 30
   Tyr Leu Ala Trp Tyr Gln Gln Lys Pro Gly Gln Ala Pro Arg Leu Leu
   35 40 45
   Ile Tyr Gly Ala Ser Ser Arg Ala Thr Gly Ile Pro Asp Arg Phe Ser
   50 55 60
   Gly Ser Gly Ser Gly Thr Asp Phe Thr Leu Thr Ile Ser Arg Leu Glu
   65 70 75 80
   Pro Glu Asp Phe Ala Val Tyr Tyr Cys Gln Gln Tyr Gly Ser Ser Phe
   85 90 95
   Arg Thr Phe Gly Gln Gly Thr Lys Gln
   100 105
<210> 17
   <211> 112
   <212> PRT
   <213> Homo sapiens
<400> 17
   Asp Ile Val Met Thr Gln Ser Pro Leu Ser Leu Ser Val Ala Pro Gly
    1               5                  10                  15
   Gln Pro Ala Ser Ile Ser Cys Lys Ser Ser Gln Ser Leu Leu Glu Ser
               20                  25                  30
   Tyr Gly Glu Thr Tyr Leu Tyr Trp Tyr Leu Gln Lys Pro Gly Gln Pro
           35                  40                  45
   Pro Gln Leu Leu Ile Tyr Ala Val Phe Lys Arg Phe Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Ser
                   85                  90                  95
   Met Gln Leu Pro Leu Thr Phe Gly Gly Gly Thr Lys Val Glu Ile Lys
               100                 105                 110
<210> 18
   <211> 108
   <212> PRT
   <213> Homo sapiens
<400> 18
   Glu Ile Val Leu Thr Gln Ser Pro Gly Thr Leu Ser Leu Ser Pro Gly
    1               5                  10                  15
   Glu Gly Ala Thr Leu Ser Cys Arg Ala Ser Gln Ser Val Ser Tyr Ser
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Ile Pro Asp Arg Phe Ser
       50                  55                  60
   Gly Ser Gly Ser Gly Thr Asp Phe Thr Leu Thr Ile Ser Arg Leu Glu
   65                  70                  75                  80
   Pro Glu Asp Phe Ala Val Tyr Tyr Cys His Gln Tyr Gly Asn Ser Phe
                   85                  90                  95
   Arg Thr Phe Gly Gln Gly Thr Lys Val Glu Ile Lys
               100                 105

## Description

### BACKGROUND OF THE INVENTION

### Field of the Invention

The invention relates to human antibodies capable of binding to CD40, methods of using the antibodies, and treatment of antibody-mediated disease in humans.

### Description of the Related Art

The CD40 antigen is a glycoprotein expressed on the cell surface of B cells and other cells, including dendritic cells. During B-cell differentiation, the molecule is first expressed on pre-B cells and then disappears from the cell surface when the B cell becomes a plasma cell. Crosslinking of the CD40 molecules with anti-CD40 antibodies mediates a variety of effects on B cells. The CD40 antigen is known to be related to the human nerve growth factor (NGF) receptor and tumor necrosis factor-α (TNF-α) receptor, suggesting that CD40 is a receptor for a ligand with important functions in B-cell activation.

CD40 is a key element of immune responses. Engagement of CD40 on antigen-presenting cells by its ligand, termed CD40L or CD154, causes production of cytokines and up-regulation of costimulatory molecules leading to efficient activation of T lymphocytes. Engagement of CD40 on B lymphocytes provides a costimulatory signal to the B cell that drives antibody production. Thus blocking of CD40 engagement and activation has the potential to suppress antibody and cell mediation immune responses. Anti-CD40 antagonist antibodies could be used to treat autoimmune disease such as systemic lupus, psoriasis, multiple sclerosis, inflammatory bowel disease (Crohn's disease), and rheumatoid arthritis. Such antibodies could also be used to prevent rejection of organ and tissue grafts by suppressing autoimmune responses, to treat lymphomas by depriving malignant B lymphocytes of the activating signal provided by CD40, and to deliver toxins to CD40-bearing cells in a specific manner.

Previously, mouse monoclonal antibodies such as 5D2 have been disclosed that bind to CD40 without providing an activating signal. These antibodies have the ability to inhibit immune responses *in vivo* and *in vitro.* However mouse antibodies cannot be used to treat human disease because they elicit human anti-mouse antibodies that hinder the effectiveness of the treatment. Therefore, there is a need in the art for antibodies of comparable specificity but composed of a human amino acid sequence.

EP0945465 describes antibodies capable of binding to CD40, methods for producing these antibodies, and methods for their use.

### BRIEF SUMMARY OF THE INVENTION

The invention provides a human monoclonal antibody or fragment thereof that is capable of specifically binding to a human CD40 antigen expressed on the surface of a normal human B cell, said monoclonal antibody or fragment being free of significant agonistic activity when said monoclonal antibody or fragment binds to the CD40 antigen expressed on the surface of said normal B cell, whereby, when said monoclonal antibody or fragment binds to the CD40 antigen expressed on the surface of said normal B cell, the growth or differentiation of said normal B cell is inhibited, wherein said monoclonal antibody or fragment thereof comprises a heavy chain variable region containing residues 31-35, 50-66, and 99-111 of SEQ ID NO:13, and comprises a light chain variable region containing residues 24-39, 55-61, and 94-102 of SEQ ID NO:17.

The invention also provides a human monoclonal antibody or fragment thereof of the invention for use in therapy.

The invention also provides a human monoclonal antibody or fragment thereof of the invention for use in (i) preventing or treating an antibody-mediated disease in a patient, (ii) preventing or treating an IgE-mediated disease in a patient, or (iii) preventing or treating an autoimmune disease in a patient.

The invention also provides the use of a human monoclonal antibody or fragment thereof of the invention in the manufacture of a medicament for (i) preventing or treating an antibody-mediated disease in a patient, (ii) preventing or treating an IgE-mediated disease in a patient, or (iii) preventing or treating an autoimmune disease in a patient.

The invention also provides a human monoclonal antibody or fragment thereof of the invention for use in inhibiting antibody production by B cells in a human patient.

The invention also provides the use of a human monoclonal antibody or fragment thereof of the invention in the manufacture of a medicament for inhibiting antibody production by B cells in a human patient.

The invention also provides a method for inhibiting growth or differentiation of a normal human B cell, or for inhibiting proliferation of a normal human B cell, wherein said proliferation is augmented by the interaction of a CD40 ligand with a CD40 antigen expressed on the surface of a B cell, said method comprising contacting said B cell *in vitro* with an effective amount of a human anti-CD40 monoclonal antibody or fragment thereof of the invention.

The invention also provides a hybridoma capable of producing the human monoclonal antibody of the invention.

The invention also provides a nucleic acid comprising a polynucleotide that encodes the human monoclonal antibody or fragment thereof of the invention, comprising the sequences set forth in SEQ ID NO:7 or SEQ ID NO: 8.

The invention also provides a pharmaceutical composition comprising the human monoclonal antibody or fragment thereof of the invention, and a pharmaceutically acceptable excipient.

Other aspects of the invention are set forth in the appended claims.

Aspects of the following description that do not relate specifically to the claimed invention are for comparison and illustration.

### BRIEF SUMMARY OF THE DISCLOSURE

Disclosed herein is a human monoclonal antibody capable of binding to a human CD40 antigen located on the surface of a human B cell, wherein the binding of the antibody to the CD40 antigen prevents the growth or differentiation of the B cell.

Also disclosed herein is a method for preventing or treating an antibody-mediated disease in a patient, the method comprising administering to a patient in need of such treatment a therapeutically effective amount of a human monoclonal antibody capable of binding to a human CD40 antigen located on the surface of a CD40-bearing cell such as a human B cell or a human dendritic cell, wherein the binding of the antibody to the CD40 antigen prevents the growth or differentiation of the cell, in a pharmaceutically acceptable excipient.

Also disclosed herein is a method for preventing or treating an IgE-mediated disease such as an allergy in a patient, the method comprising administering to a patient in need of such treatment a therapeutically effective amount of a human monoclonal antibody capable of binding to a human CD40 antigen located on the surface of a human B cell, wherein the binding of the antibody to the CD40 antigen prevents the growth or differentiation of the B cell, in a pharmaceutically acceptable excipient.

Also disclosed herein is a method for preventing or treating an autoimmune disease in a patient, including an antibody-mediated disease, the method comprising administering to a patient in need of such treatment a therapeutically effective amount of a human monoclonal antibody capable of binding to a human CD40 antigen located on the surface of a human B cell, wherein the binding of the antibody to the CD40 antigen prevents the growth or differentiation of the B cell, in a pharmaceutically acceptable excipient. Particular autoimmune diseases contemplated for treatment by this method include systematic lupus erythematosus (SLE), primary biliary cirrhosis (PBC), and idiopathic thrombocytopenic purpura (ITP).

Also disclosed herein is a method of inhibiting growth of tumor cells, including Non-Hodgkins Lymphoma.

In more preferred embodiments of the above disclosure, the monoclonal antibody is 15B8, 20C4, 13E4, 12D9, or 9F7.

### BRIEF DESCRIPTION OF THE SEVERAL VIEWS OF THE DRAWINGS

Figure 1 is a bar graph illustrating the effect of anti-CD40 antibodies on proliferation of B cells stimulated by CD40L-expressing cells.
Figure 2 is a bar graph illustrating the effect of anti-CD40 antibodies on B cell proliferation.
Figure 3 is a bar graph illustrating the effect of CD40 antibodies on anti-IgM-induced B cell proliferation.
Figure 4 is a bar graph illustrating the effect of crosslinked anti-CD40 antibody on proliferation of human peripheral B cells.
Figure 5 is a comparison of the amino acid sequences of the light chains of five human anti-CD40 antibodies and 5H7.
Figure 6 is a comparison of the amino acid sequences of the heavy chains of five human anti-CD40 antibodies and 5H7.
Figure 7 shows the results of FACS analysis of monoclonal antibody binding to cells expressing CD40, 15B8, showing that the antibody stained peripheral blood cells from three species: humans; Rhesus; and cynomologus macaques.
Figure 8 provides the DNA and amino acid sequences for the vK region of human monoclonal antibody 12D9
Figure 9 provides the DNA and amino acid sequences for the heavy chain constant region of human monoclonal antibody 12D9, SEQ ID NOs:1 and 2, respectively.
Figure 10 provides the DNA sequences for the vK.1 and vh1 regions of human monoclonal antibody 20C4, SEQ ID NOs:3 and 4, respectively.
Figure 11 provides the DNA sequences for the vK.1 and vh1 regions of human monoclonal antibody 9F7, SEQ ID NOs:5 and 6, respectively.
Figure 12 provides the DNA sequences for the vK.3 and vh1 regions of human monoclonal antibody 15B8, SEQ ID NOs:7 and 8, respectively.
Figure 13 provides the DNA sequence for the vh1 regions of human monoclonal antibodies 13E4 and 12D9 (SEQ ID NO:10).
Figure 14 provides the amino acid sequences for the following regions of the indicated human monoclonal antibodies:
   9F7VH1: SEQ ID NO:11
   12D9VH1: SEQ ID NO:12
   15B8VH1 SEQ ID NO:13
   20C4VH1: SEQ ID NO:14
   9F7VK1: SEQ ID NO:15
   12D9VK1: SEQ ID NO:16
   15B8VK1: SEQ ID NO:17
   20C4VK1: SEQ ID NO:18
Figure 15 shows that anti-CD40 antibody MS81 stimulated proliferation of NHL cells in the presence and absence of IL-4, using cells from one patient.
Figure 16 shows that anti-CD40 antibody MS81 stimulated proliferation of NHL cells in the presence and absence of IL-4, using cells from a second patient.
Figure 17 shows that anti-CD40 antibody 15B8 inhibits the proliferation of NHL cells in one patient.
Figure 18 shows the dose response to 15B8 in proliferation of NHL cells from a rituxan-sensitive patient. The cells were stimulated with CD40L and IL-4.
Figure 19 shows representative dose-response curves for 15B8 effect on proliferation of human B cells stimulated by CD40L using cells from three individuals.

### DETAILED DESCRIPTION

Antibodies are constructed of several regions, a crucial region being the complementarity determining region, or CDR. The phrase "complementarity determining region" refers to amino acid sequences which together define the binding affinity and specificity of the natural Fv region of a native immunoglobulin binding site. *See, e.g.,* Chothia et al., J. Mol. Biol. 196:901-917 (1987); Kabat et al., U.S. Dept. of Health and Human Services NIH Publication No. 91-3242 (1991). The phrase "constant region" refers to the portion of the antibody molecule that confers effector functions. In previous work directed towards producing non-immunogenic antibodies for use in therapy of human disease, mouse constant regions were substituted by human constant regions. The constant regions of the subject humanized antibodies were derived from human immunoglobulins. However, these humanized antibodies still elicited an unwanted and potentially dangerous immune response in humans and there was a loss of affinity.

The human monoclonal anti-CD40 antibodies of the present disclosure address the shortcomings of prior art monoclonal antibodies. Accordingly, the human monoclonal antibodies disclosed herein preferably produced using transgenic animals that are engineered to contain human immunoglobulin loci. For example, WO 98/24893 discloses transgenic animals having a human Ig locus wherein the animals do not produce functional endogenous immunoglobulins due to the inactivation of endogenous heavy and light chain loci. WO 91/10741 also discloses transgenic non-primate mammalian hosts capable of mounting an immune response to an immunogen, wherein the antibodies have primate constant and/or variable regions, and wherein the endogenous immunoglobulin-encoding loci are substituted or inactivated. WO 94/02602 discloses non-human mammalian hosts having inactivated endogenous Ig loci and functional human Ig loci. U.S. Patent No. 5,939,598 discloses methods of making transgenic mice in which the mice lack endogenous heavy claims, and express an exogenous immunoglobulin locus comprising one or more xenogeneic constant regions.

Using a transgenic animal described above, an immune response can be produced to a selected antigenic molecule, in this case CD40, and antibody-producing cells can be removed from the animal and used to produce hybridomas that secrete human monoclonal antibodies. Immunization protocols, adjuvants, and the like are known in the art, and are used in immunization of, for example, a transgenic mouse as described in WO 96/33735. The monoclonal antibodies can be tested for the ability to inhibit or neutralize the biological activity or physiological effect of the corresponding protein.

As used herein, the term "antibody" refers to polyclonal antibodies, monoclonal antibodies, single-chain antibodies, and fragments thereof such as Fab, F(ab')₂, Fᵥ, and other fragments which retain the antigen binding function of the parent antibody.

As used herein, the term "monoclonal antibody" refers to an antibody composition having a homogeneous antibody population. The term is not limited regarding the species or source of the antibody, nor is it intended to be limited by the manner in which it is made. The term encompasses whole immunoglobulins as well as fragments such as Fab, F(ab')₂, Fᵥ, and others which retain the antigen binding function of the antibody. Monoclonal antibodies of any mammalian species can be used in this disclosure. In practice, however, the antibodies will typically be of rat or murine origin because of the availability of rat or murine cell lines for use in making the required hybrid cell lines or hybridomas to produce monoclonal antibodies.

As used herein, the term "single chain antibodies" refer to antibodies prepared by determining the binding domains (both heavy and light chains) of a binding antibody, and supplying a linking moiety which permits preservation of the binding function. This forms, in essence, a radically abbreviated antibody, having only that part of the variable domain necessary for binding to the antigen. Determination and construction of single chain antibodies are described in U.S. Pat. No. 4,946,778 to Ladner et al.

The term "CD40 antigen epitope" as used herein refers to a molecule which is capable of immunoreactivity with the anti-CD40 monoclonal antibodies of this invention, excluding the CD40 antigen itself. CD40 antigen epitopes may comprise proteins, protein fragments, peptides, carbohydrates, lipids, and other molecules, but for the purposes of the present disclosure are most commonly proteins, short oligopeptides, oligopeptide mimics (*i.e.,* organic compounds which mimic the antibody binding properties of the CD40 antigen), or combinations thereof. Suitable oligopeptide mimics are described, *inter alia,* in International publication No. WO91/19735

The antibodies of the current disclosure are produced by transgenic mice bearing human immunoglobulin loci, and bind to a human CD40 antigen on the surface of a human cell, particularly a B cell. These antibodies may be polyclonal antibodies, monoclonal antibodies, single-chain antibodies, and fragments thereof.

Monoclonal antibodies 15B8, 20C4, 13E4, 12D9, and 9F7 are prepared as described in the Examples. Other antibodies may be prepared similarly using mice transgenic for human immunoglobulin loci or by other methods known in the art and/or described herein.

Polyclonal sera may be prepared by conventional methods. In general, a solution containing the CD40 antigen is first used to immunize a suitable animal, in the present disclosure a transgenic animal, preferably a mouse bearing human immunoglobulin loci. In a preferred embodiment, Sf9 cells expressing CD40 are used as the immunogen. Immunization can also be performed by mixing or emulsifying the antigen-containing solution in saline, preferably in an adjuvant such as Freund's complete adjuvant, and injecting the mixture or emulsion parenterally (generally subcutaneously or intramuscularly). A dose of 50-200 µg/injection is typically sufficient. Immunization is generally boosted 2-6 weeks later with one or more injections of the protein in saline, preferably using Freund's incomplete adjuvant. One may alternatively generate antibodies by *in vitro* immunization using methods known in the art, which for the purposes of this disclosure is considered equivalent to *in vivo* immunization.

Polyclonal antisera are obtained by bleeding the immunized animal into a glass or plastic container, incubating the blood at 25°C for one hour, followed by incubating at 4°C for 2-18 hours. The serum is recovered by centrifugation (*e.g*., 1,000 x g for 10 minutes). About 20-50 ml per bleed may be obtained from rabbits.

Monoclonal antibodies can be prepared using the method of Kohler and Milstein, Nature 256:495-96 (1975), or a modification thereof. Typically, a mouse is immunized as described above. However, rather than bleeding the animal to extract serum, the spleen (and optionally several large lymph nodes) are removed and dissociated into single cells. If desired, the spleen cells may be screened (after removal of nonspecifically adherent cells) by applying a cell suspension to a plate or well coated with the protein antigen. B-cells expressing membrane-bound immunoglobulin specific for the antigen bind to the plate, and are not rinsed away with the rest of the suspension. Resulting B-cells, or all dissociated spleen cells, are then induced to fuse with myeloma cells to form hybridomas, and are cultured in a selective medium (*e.g*., hypoxanthine, aminopterin, thymidine medium, "HAT"). The resulting hybridomas are plated by limiting dilution, and are assayed for the production of antibodies which bind specifically to the desired immunizing cell-surface antigen (and which do not bind to unrelated antigens). The selected MAb-secreting hybridomas are then cultured either *in vitro* (*e.g.,* in tissue culture bottles or hollow fiber reactors), or *in vivo* (as ascites in mice).

As an alternative to use of hybridomas, antibody can be produced in a cell line such as a CHO cell line, as disclosed in U.S. Patent Nos. 5,545,403, 5,545,405, and 5,998,144. Briefly, the cell line is transfected with vectors capable of expressing a light chain and a heavy chain, respectively. By transfecting the two proteins on separate vectors, chimeric antibodies can be produced. Another advantage is the correct glycosylation of the antibody.

Preferably, fully human antibodies to CD40 are obtained by immunizing transgenic mice. One such mouse is referred to as a Xenomouse, and is disclosed in U.S. Patent Nos. 6,075,181; 6,091,001; and 6,114,598. To produce the antibodies disclosed herein, mice transgenic for the human IgG2 heavy chain locus and the human K light chain locus were immunized with Sf9 cells expressing human CD40. Mice can also be transgenic for other isotypes.

Production of the Sf9 (*Spodoptera frugiperda*) cells is disclosed in de Boer, U.S. Patent No. 6,004,552. Briefly, sequences encoding human CD40 were recombined into a baculovirus using transfer vectors as described by de Boer. The plasmids were co-transfected with wild-type baculovirus DNA into Sf9 cells. Recombinant baculovirus-infected Sf9 cells were identified and clonally purified.

Mice were injected intraperitoneally (IP) at day 0 and day 14 with 5 x 10⁶ Sf9 cells expressing CD40. A final injection was made at least five weeks later, and the spleen and thymus cells were removed and used for cell fusion. Cell fusion was carried out as described by de Boer. Hybridoma antibodies were screened as described in the Examples. Five hybridomas were selected for further study, based on their ability to inhibit proliferation of human peripherial blood B cells induced by CD40 ligand (CD40L) and anti-IgM, and their ability to inhibit production of IgM by human peripheral blood B cells stimulated with anti-CD3-activated human peripheral blood T cells.

The five hybridomas showing the optimal inhibitory activity were designated 15B8.8.6 (15B8), 20C4.1.6 (20C4), 13E4.12.11 (13E4), 12D9.9.10 (12D9), 9F7.9.11.1 (9F7), and 15B8.7.2. None of these hybridomas showed significant ability to induce proliferation in resting human peripheral blood B cells.

The relative binding properties of these hybridoma antibodies was examined by flow cytometry, as described in detail in the Examples. Briefly, the antibodies compared exhibited differences in affinity despite their ability to recognize the same or closely related epitopes. For example, MAb 15B8 blocked binding of MAb 20C4 to human CD20⁺ peripheral blood lymphocytes, but MAb 20C4 did not block MAb 15B8 binding to the CD20⁺ lymphocytes. The differential CD40 binding of hybridomas is shown in Table 4 (Example 4).

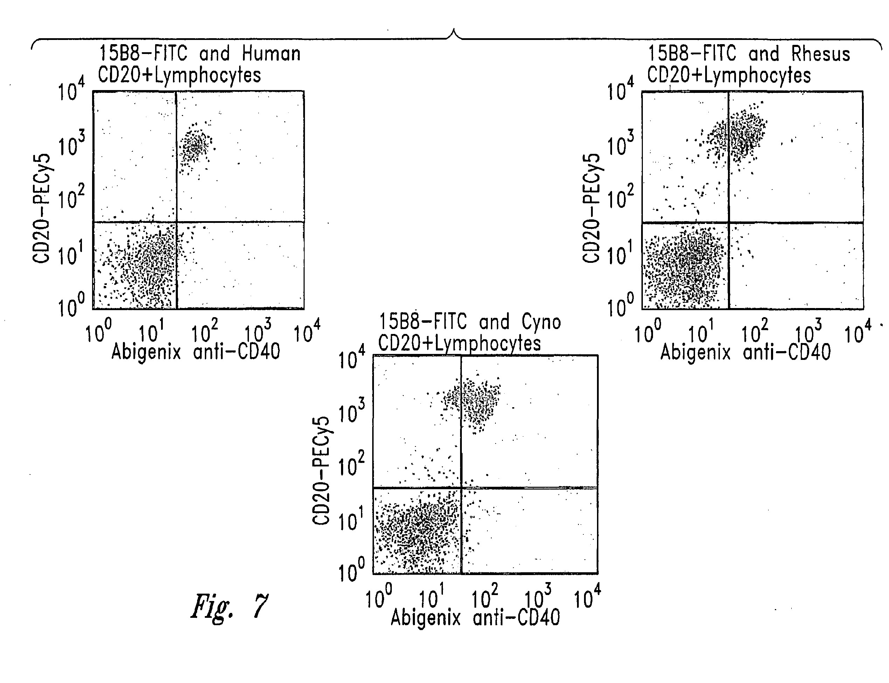

Four hybridomas tested (15B8, 20C4, 12D9, and 9F7) produced monoclonal antibodies that stained peripheral blood cells from three species: humans; Rhesus; and cynomologus macaques (Figure 7).

To determine the polynucleotide sequences encoding the monoclonal antibodies, mRNA was prepared from the hybridomas and RT-PCR was performed on the mRNAs using standard procedures. The PCR products were analyzed on gels, sequenced, and translated. The polynucleotide and amino acid sequences are provided in SEQ ID NOs:1-18, as shown in Table 7, Example 11.

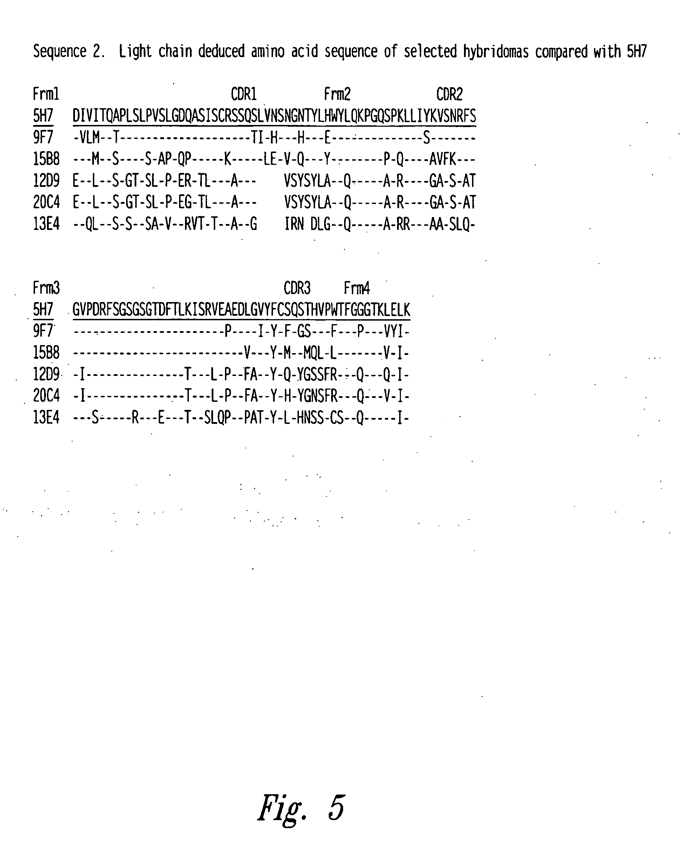

The amino acid sequences of five monoclonal antibodies (9F7, 15B8, 12D9, 20C4, and 13E4) were compared with the amino acid sequence of mouse anti-CD40 monoclonal antibody 5H7, as shown in Figures 5 (light chains) and 6 (heavy chains).

The results obtained using the five disclosed monoclonal antibodies indicate that these antibodies, as well as fragments and chimeric forms thereof, have antagonistic features that make them suitable for a number of clinical applications, including treatment of autoimmune diseases, treatment of transplantation reactions and rejections, as adjuvant therapies for gene therapies and protein therapies, and in inhibiting growth of tumor cells, including Non-Hodgkins Lymphoma cells. Additional uses include treatment of any disease mediated by a CD40-expressing malignant cell, and use to treat diseases related to the proliferation, activation, or regulation of cells expressing CD40. The activity of the five MAb's is summarized in Table 1.

**Table 1**

| **Clones** | **Inhibition on IgM Secretion of B cells** | **Inhibition on proliferatio n of Jurkat cell-stimulated B cells** | **Inhibition on proliferation of CHO-CD40L cell-stimulated B cells** | **Stimulation on proliferation of anti-higM-stimulated B cells** | **Stimulatio n on proliferati on of B cells** | **Ab cross-linking to stimulate the proliferation of B cells** |
|---|---|---|---|---|---|---|
| MS81 12D9.9.10 | -29% | -64% | -24% | 190% | 178% | 120% |
| MS81 15B8.8.6.12 | -47% | -81% | -89% | 352% | 513% | 76% |
| MS81 20C4.1.6 | -35% | -74% | -97% | 237% | 279% | 143% |
| MS81 13E4.12.11 | -44% | -50% | n/a | 346% | 660% | n/a |
| MS81 9F7.9.11.1 | -30% | -71% | -57% | 120% | 275% | 84% |

| | | | | | | |
|---|---|---|---|---|---|---|
| * representative data from the antibodies at 1µg/ml were shown | | | | | | |

The disclosure encompasses not only the five monoclonal antibodies described herein, but also antibodies differing from these but retaining the CDR; and antibodies with one or more amino acid addition(s), deletion(s), or substitution(s), wherein the activity is measured by inhibition of B cell proliferation and/or antibody secretion. The disclosure also encompasses de-immunized antibodies, which can be produced as described in, for example, WO 98/52976, "Method for the Production of Non-Immunogenic Proteins," Also disclosed herein are fusion proteins comprising a monoclonal antibody of the disclosure, or a fragment thereof, which fusion proteins can be synthesized or expressed from corresponding polynucleotide vectors, as is known in the art.

The antibodies of the present disclosure can have sequence variations produced using methods described in, for example, Patent Publication Nos. EP 0 983 303 A1, WO 00/34317, and WO 98/52976 . For example, it has been shown that sequences within the CDR can cause an antibody to bind to MHC Class II and trigger an unwanted helper T cell response. A conservative substitution can allow the antibody to retain binding activity yet lose its ability to trigger an unwanted T cell response. Any such conservative or non-conservative substitutions can be made using art-recognized methods, and the resulting antibodies will fall within the scope of the disclosure.

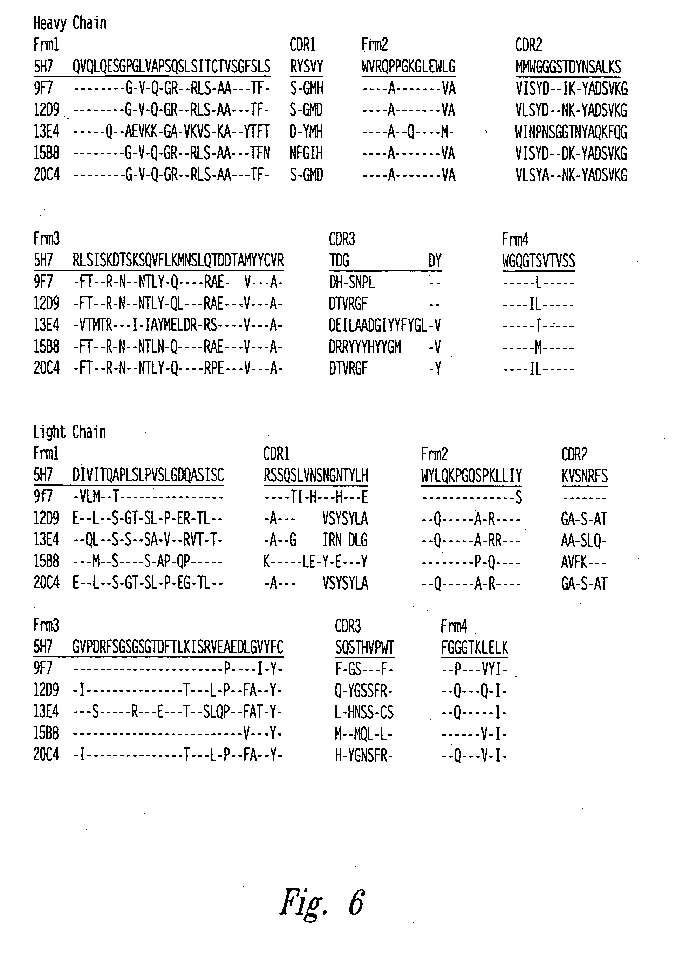

Also disclosed herein are amino acid sequences for light chains and heavy chains of the preferred monoclonal antibodies (SEQ ID NOS. 1-18). The sequences are aligned as shown in Figures 5 and 6. These alignments indicate specific amino acid positions that are more amenable to substitution without loss of the desired biological activities of the antibody. Using only routine methods, one of skill in the art can construct plasmids that will encode variants of these sequences. The variant antibodies can be routinely tested for antagonistic activity, affinity, specificity, and agonistic activity using methods described herein.

An antibody produced by any of the methods described above, or any other method not disclosed herein, will fall within the scope of this disclosure if it possesses at least one of the following biological activities: inhibition of immunoglobulin secretion by human peripheral B cells stimulated by T cells; inhibition of proliferation of human peripheral B cells stimulated by Jurkat T cells; and inhibition of proliferation of human peripheral B cells stimulated by CD40L-expressing cells. These assays can be performed as described in the Examples herein.

The antibodies will also exhibit a single site binding affinity (K_{D}) of at least 10⁻⁵ M, preferably at least 10⁻⁶ - 10⁻⁷ M, more preferably at least 10⁻⁸ M, and most preferably at least 10⁻⁹ M, such as 10⁻¹⁰ M, as measured using a standard assay such as Biacore, which is know in the art, in comparison with appropriate controls. Binding affinity of 10⁻¹¹ M, 10⁻¹³ M, 10⁻¹⁵ M, 10⁻¹⁷ M and 10⁻¹⁹ M can also be achieved. These assays are automated, and allow the measurement of MAb specificity and cross-reactivity, which can also be assayed using standard techniques known in the art. Details of the Biacore assays are provided in Biacore's "BIAapplications handbook." Methods described in WO 01/27160 can be used to modulate the binding affinity.

If desired, the antibodies (whether polyclonal or monoclonal) may be labeled using conventional techniques. Suitable labels include fluorophores, chromophores, radioactive atoms (particularly ³²P and ¹²⁵I), electron-dense reagents, enzymes, and ligands having specific binding partners. Enzymes are typically detected by their activity. For example, horseradish peroxidase is usually detected by its ability to convert 3,3',5,5'-tetramethylbenzidine (TMB) to a blue pigment, quantifiable with a spectrophotometer. "Specific binding partner" refers to a protein capable of binding a ligand molecule with high specificity, as for example in the case of an antigen and a monoclonal antibody specific therefor. Other specific binding partners include biotin and avidin or streptavidin, IgG and protein A, and the numerous receptor-ligand couples known in the art. It should be understood that the above description is not meant to categorize the various labels into distinct classes, as the same label may serve in several different modes. For example, ¹²⁵I may serve as a radioactive label or as an electron-dense reagent. HRP may serve as enzyme or as antigen for a MAb. Further, one may combine various labels for desired effect. For example, MAbs and avidin also require labels in the practice of this invention: thus, one might label a MAb with biotin, and detect its presence with avidin labeled with ¹²⁵I, or with an anti-biotin MAb labeled with HRP. Other permutations and possibilities will be readily apparent to those of ordinary skill in the art, and are considered as equivalents within the scope of the instant disclosure.

Antibodies for use in the disclosure can be produced using any suitable technique. For example, WO 01/27160 discloses a method of conferring donor CDR binding affinity onto an antibody acceptor variable region framework. The method can also be used to optimize the binding affinity of a variable region or an antibody, such as to enhance the binding affinity. Methods for producing humanized antibodies having one or more CDR's are disclosed in U.S. Patent No. 6,180,370. Methods of producing antibodies that have been optimized for administration to humans are disclosed in WO 00/34317, which describes the production of proteins that are rendered less immunogenic or non-immunogenic. U.S. Patent No. 5,514,548 discloses methods for selection of ligand binding proteins, such as antibodies, that bind with high affinity to a target ligand. U.S. Patent No. 5,877,397 disclosed transgenic non-human animals capable of producing heterologous antibodies.

### FORMULATIONS AND METHODS OF ADMINISTRATION

The antibodies of this disclosure are administered at a concentration that is therapeutically effective to prevent or treat antibody-mediated diseases such as allergies, SLE, PBC, ITP, multiple sclerosis, psoriasis, Crohn's disease, graft rejection, and B cell lymphoma. To accomplish this goal, the antibodies may be formulated using a variety of acceptable excipients known in the art. Typically, the antibodies are administered by injection, either intravenously or intraperitoneally. Methods to accomplish this administration are known to those of ordinary skill in the art. It may also be possible to obtain compositions which may be topically or orally administered, or which may be capable of transmission across mucous membranes.

Before administration to patients, formulants may be added to the antibodies. A liquid formulation is preferred. For example, these formulants may include oils, polymers, vitamins, carbohydrates, amine acids, salts, buffers, albumin, surfactants, or bulking agents. Preferably carbohydrates include sugar or sugar alcohols such as mono-, di-, or polysaccharides, or water soluble glucans. The saccharides or glucans can include fructose, dextrose, lactose, glucose, mannose, sorbose, xylose, maltose, sucrose, dextran, pullulan, dextrin, alpha and beta cyclodextrin, soluble starch, hydroxethyl starch and carboxymethylcellulose, or mixtures thereof. Sucrose is most preferred. "Sugar alcohol" is defined as a C₄ to C₈ hydrocarbon having an --OH group and includes galactitol, inositol, mannitol, xylitol, sorbitol, glycerol, and arabitol. Mannitol is most preferred. These sugars or sugar alcohols mentioned above may be used individually or in combination. There is no fixed limit to amount used as long as the sugar or sugar alcohol is soluble in the aqueous preparation. Preferably, the sugar or sugar alcohol concentration is between 1.0 w/v % and 7.0 w/v %, more preferable between 2.0 and 6.0 w/v %. Preferably amino acids include levorotary (L) forms of carnitine, arginine, and betaine; however, other amino acids may be added. Preferred polymers include polyvinylpyrrolidone (PVP) with an average molecular weight between 2,000 and 3,000, or polyethylene glycol (PEG) with an average molecular weight between 3,000 and 5,000. It is also preferred to use a buffer in the composition to minimize pH changes in the solution before lyophilization or after reconstitution. Most any physiological buffer may be used, but titrate, phosphate, succinate, and glutamate buffers or mixtures thereof are preferred. Most preferred is a citrate buffer. Preferably, the concentration is from 0.01 to 0.3 molar. Surfactants that can be added to the formulation are shown in EP Nos. 270,799 and 268,110.

Additionally, antibodies can be chemically modified by covalent conjugation to a polymer to increase their circulating half-life, for example. Preferred polymers, and methods to attach them to peptides, are shown in U.S. Pat. Nos. 4,766,106; 4,179,337; 4,495,285; and 4,609,546.

Preferred polymers are polyoxyethylated polyols and polyethylene glycol (PEG). PEG is soluble in water at room temperature and has the general formula: R(O--CH₂ --CH₂)ₙ O--R where R can be hydrogen, or a protective group such as an alkyl or alkanol group. Preferably, the protective group has between 1 and 8 carbons, more preferably it is methyl. The symbol n is a positive integer, preferably between 1 and 1,000, more preferably between 2 and 500. The PEG has a preferred average molecular weight between 1000 and 40,000, more preferably between 2000 and 20,000, most preferably between 3,000 and 12,000. Preferably, PEG has at least one hydroxy group, more preferably it is a terminal hydroxy group. It is this hydroxy group which is preferably activated to react with a free amino group on the inhibitor. However, it will be understood that the type and amount of the reactive groups may be varied to achieve a covalently conjugated PEG/antibody of the present invention.

Water soluble polyoxyethylated polyols are also useful in the present invention. They include polyoxyethylated sorbitol, polyoxyethylated glucose, polyoxyethylated glycerol (POG), etc. POG is preferred. One reason is because the glycerol backbone of polyoxyethylated glycerol is the same backbone occurring naturally in, for example, animals and humans in mono-, di-, triglycerides. Therefore, this branching would not necessarily be seen as a foreign agent in the body. The POG has a preferred molecular weight in the same range as PEG The structure for POG is shown in Knauf et al., J. Bio. Chem. 263:15064-15070 (1988), and a discussion of POG/IL-2 conjugates is found in U.S. Pat. No. 4,766,106.

Another drug delivery system for increasing circulatory half-life is the liposome. Methods of preparing liposome delivery systems are discussed in Gabizon et al., Cancer Research 42:4734 (1982); Cafiso, Biochem Biophys Acta 649:129 (1981); and Szoka, Ann Rev Biophys Eng 9:467 (1980). Other drug delivery systems are known in the art and are described in, *e.g.*, Poznansky et al., Drug Delivery Systems (R. L. Juliano, ed., Oxford, N.Y. 1980), pp. 253-315; M. L. Poznansky, Pharm Revs 36:277 (1984).

After the liquid pharmaceutical composition is prepared, it is preferably lyophilized to prevent degradation and to preserve sterility. Methods for lyophilizing liquid compositions are known to those of ordinary skill in the art. Just prior to use, the composition may be reconstituted with a sterile dilutent (Ringer's solution, distilled water, or sterile saline, for example) which may include additional ingredients. Upon reconstitution, the composition is preferably administered to subjects using those methods that are known to those skilled in the art.

As stated above, the antibodies and compositions of this invention may be used to treat human patients to prevent or treat antibody-mediated diseases such as allergies, SLE, PBC and ITP. The preferred route of administration is parenterally. In parenteral administration, the compositions of this invention will be formulated in a unit dosage injectable form such as a solution, suspension or emulsion, in association with a pharmaceutically acceptable parenteral vehicle. Such vehicles are inherently nontoxic and nontherapeutic. Examples of such vehicles are saline, Ringer's solution, dextrose solution, and Hanks' solution. Nonaqueous vehicles such as fixed oils and ethyloleate may also be used. A preferred vehicle is 5% dextrose in saline. The vehicle may contain minor mounts of additives such as substances that enhance isotonicity and chemical stability, including buffers and preservatives.

The dosage and mode of administration will depend on the individual. Generally, the compositions are administered so that antibodies are given at a dose between 1 µg/kg and 20 mg/kg, more preferably between 20 µg/kg and 10 mg/kg, most preferably between 1 and 7 mg/kg. Suitably, it is given as an infusion or as a bolus dose, to increase circulating levels by 10-20 fold and for 4-6 hours after the bolus dose. Continuous infusion may also be used after the bolus dose. If so, the antibodies may be infused at a dose between 5 and 20 µg/kg/minute, more preferably between 7 and 15 µg/kg/minute. Suitable treatment regimens are disclosed in WO 00/27428 and WO 00/27433.

### Use of Anti-CD40 Antibody Therapy for Non-Hodgkins Lymphoma Therapy

Non-Hodgkin's Lymphoma (NHL) originates from components of the spleen, thymus and lymph nodes (Jandl J.H., Non-Hogkin's lymphomas, in Jandl JH (ed): Blood, Textbook of Hematology, Boston, MA, Little Brown, 1996, pp. 853-887). It consists of a group of lymphocytic malignancies that are derived primarily from B and T cells. Patients with low grade NHL are usually non-responsive to radiation therapy and chemotherapy. This low response rate and the high probability of relapse contribute to the median patient survival time of fewer than 9 years.

The imbalance of growth/survival signal by CD40 and crippled death signal by Fas plays an important role in the pathogenesis of low grade B lineage malignancies, including chronic lymphcytic leukemia (CLL) and NHL (Ghia P., Adv. Cancer Res., 2000, 79:157-73). Studies in low grade NHL suggests the disease onset is due to the accumulation of the lymphoma cells as a results of reduction in apoptosis through the Fas pathway and increase in the survival signal through CD40 (Ghia P., Adv. Cancer Res., 2000, 79:157-73). This may explain the insensitivity of the lymphoma cells to chemo or radiation therapy, which specifically target actively proliferating cells.

The disclosure further relates to a new NHL therapy comprising the use of an antibody to CD40 to block the survival signal for the NHL cells. This strategy is supported by a number of observations in the published scientific literature. CD40 is expressed on the surface of B cells throughout B-cell development. Studies have demonstrated that CD40 provides a survival signal for malignant B cells and stimulates their growth *in vitro* (Romano M.F. et al., Leuk Lymphoma, 2000 Jan., 36(3-4):255-62; Furman R.R., J. Immunol., 2000 Feb. 15, 164(4):2200-6; Kitada S., Br. J. Haematol., 1999 Sep., 106(4):995-1004; Romano M.F., Blood, 1998 Aug. 1, 92(3):990-5; Jacob A., Leuk. Res., 1998 Apr., 22(4):379-82; Wang D., Br. J. Haematol., 1997 May, 97(2):409-17; Planken E.V., Leukemia, 1996 Mar., 10(3):488-93; Greiner A., Am. J. Pathol., 1997 May, 150(5):1583-93). There is evidence from patients that the microenvironment exists to provide CD40L for CD40 signaling in vivo: CD40 is expressed on lymphoma cells in 86% of patients with B-lineage NHL (Uckun F.M., Blood, 1990 Dec. 15, 76(12):2449-56). The discovery of CD40/CD40L co-expression in the same B-cell lymphoma cells raises the possibility of an autocrine growth signal loop in NHL patients (Clodi K., Br. J. Haematol., 1998 Oct., 103(1):270-5). There is also a significant increase in soluble CD40L in the NHL patient serum (Younes A., Br. J. Haematol., 1998 Jan., 100(1):135-41). The soluble CD40L can induce proliferation of lymphoma cells in primary NHL lymphoma cell culture (Andersen N.S., Blood, 2000 Sep. 15, 96(6):2219-25; Buske C., Leukemia, 1997 Nov., 11(11):1862-7). CD40L expression is increased in the tumor marginal zone in low-grade MALT lymphomas (Carbone A., Am. J. Pathol., 1995 Oct., 147(4):912-22; Greiner A., Dev. Immunol., 1998, 6(3-4):17-95). Given the high level of CD40 expressed on tumors of B-cell lineage and its function as a survival signal for these malignant cells, an antagonist anti-CD40 antibody may have therapeutic value in NHL.

15B8, a human IgG2 subtype anti-human CD40 monoclonal antibody generated by immunization of transgenic mice bearing the human IgG2 heavy chain demonstrate the potential efficacy of 15B8 in a preclinical *in vitro* model of NHL, 15B8 was tested using malignant B cells (NHL cells) obtained from NHL patients who were either rituximab treated or naïve. Rituximab is an anti-CD20 monoclonal antibody for the treatment of relapsed or refractory low grade or follicular NHL.

Since primary lymphoma cells do not proliferate in regular culture medium and undergo apoptosis after a few days in culture, tumor cells were co-cultured with irradiated CD40-ligand (CD40L) transfected feeder cells (Arpin, C., Science, 1995, 268:720-722) in the presence or absence of the B cell growth factor Interleukin-4 (IL-4). Antibodies (agonist anti-CD40 MS81, or antagonist anti-CD40 15B8 or isotype control huIgG2) of indicated concentration (from 0.01 µg/ml to 10 µg/ml) were then added to the culture. Following incubation at 37°C for 48 hours, cultured cells were pulsed with ³H-thymidine for 18 hours. The cells were then harvested and analyzed for the amount of ³H-thymidine incorporation (Schultz, J.L., Proc. Natl. Acad. Sci. USA, 1995, 92:8200-8204). All sample conditions were in triplicate.

In these NHL cell primary culture assays 15B8 alone or in combination with IL-4 did not stimulate NHL cells to proliferate *in vitro.* In contrast, an agonist anti-CD40 MS81 induced NHL cell proliferation under the same condition. 15B8 showed statistically significant inhibition of NHL cell proliferation stimulated by CD40L (P=0.05) and by CD40L plus IL-4 (P<0.05) *in vitro.* At 1-10 µg/ml or 0.1-10 µg/ml concentration range respectively, 15B8 showed a statistically significant dose related inhibition of NHL cell proliferation stimulated by CD40L or by CD40L plus IL-4 (P<0.005).

There are two types of preclinical models that are currently used for evaluation of human antigen-specific Mabs in therapeutic development for lymphomas. One model is the xenograft mouse *in vivo* model, where the EBV-transformed lymphoma cell lines, such as Daudi (Burkitt lymphoma) or Raji (Burkitt lymphoma) cells, are xenografted into SCID/Nude mice. However, in these models, the results only reflect effects on the particular immortal cell line, which is derived from one EBV-transformed cell. It is known that Burkitt lymphoma cells are lymphoblastoid cells (Ambinder R.F., Cancer Treat. Res., 1999, 99:27-45; Quintanilla-Martinez L., Leuk. Lymphoma, 1998 Jun., 30(1-2):111-21; Klein G., Acta Microbiol. Immunol. Hung., 1996, 43(2-3):97-105) while the lymphoma cells from NHL patients are believed to be at the mature B cell stage (Ghia P., Adv. Cancer Res., 2000, 79:157-73). EBV transformation of B cells results in changes of many components in the CD40 signaling pathway (Uchida J., Science, 1999 Oct. 8, 286(5438):300-3; Farrell P.J., Biomed. Pharmacother., 1997, 51(6-7):258-67). In contrast to CD40 signaling in NHL cells and normal B cells, CD40 signaling leads to growth arrest in EBV-transformed Burkitt lymphoma cell lines (Fukuda M., Viral Immunol., 2000, 13(2):215-29; Baker M.P., Blood, 1998 Oct. 15, 92(8):2830-43). Thus, the results of testing an antagonist anti-CD40 MAb (15B8) in the xenograft models will not be able to predict the response to the antibody (15B8) by NHL patients.

The other model is the *in vitro* growth inhibition assay of lymphomas cells from NHL patients, which was used herein. The advantage is that the results predicate the sensitivity of the lymphoma cells from NHL patients to the agent (15B8) tested. However, the results are obtained from *in vitro* study under defined conditions. A previously published study reported that a rat anti-mouse CD40, which failed to induce ADCC and CDD *in vitro,* showed good efficacy in two syngeneic mouse B lymphoma models (BCL1 and A31) (Tutt A.L., J. Immunol., 1998 Sep. 15, 161(6):3176-85). The anti-tumor effect on the anti-mouse CD40 occurred slower in time than an anti-Id tested. The anti-mouse CD40 may operate by blocking critical growth signals that are dependent on the expression of surface CD40 not direct signaling like anti-Id in the mouse models tested. This study suggests that the blocking of CD40/CD40L signaling by an anti-CD40 could be efficacious *in vivo.* When tested, 15B8 did not bind to the Fcγ receptors *in vitro* and failed to induce ADCC and CDC *in vitro* since it is of human IgG2 subtype. 15B8 is of similar property to the rat anti-mouse CD40. This data supports the hypothesis that 15B8 will be beneficial to NHL patients, especially Rituxan-resistant patients.

### EXAMPLES

### GENERAL METHODS

### ELISA Assay for Immunoglobulin Quantification

The concentrations of human IgM and IgG were estimated by ELISA. 96-well ELISA plates were coated with 2 µg/ml goat anti-human IgG MAb (The Jackson Laboratory, Bar Harbor, Maine) or with 2 µg/ml goat anti-human IgM MAb 4102 (BioSource International, Calif.) in 0.05M carbonate buffer (pH 9.6), by incubation for 16 hours at 4°C. Plates were washed 3 times with PBS-0.05% Tween-20 (PBS-Tween) and saturated with BSA for 1 hour. After 2 washes the plates were incubated for 2 hour at 37°C with different dilutions of the test samples. After 3 washes, bound Ig was detected by incubation for 2 hour at 37°C with 1 µg/ml peroxidase-labeled goat anti-human IgG MAb or goat anti-human IgM MAb. Plates were washed 4 times and bound peroxidase activity was revealed by the addition of O-phenylenediamine as a substrate. Human IgG or IgM standards (Caltaq, Burlingame, CA) was used to establish a standard curve for each assay.

### Isolation of the Peripheral Blood Mononuclear Cells (PBMC) from Human Peripheral Blood.

20ml of Ficoll-Paque solution (low endotoxin, Pharmacia) was added per 50 ml polystyrene tube, in 3 tubes, 30 minutes before adding the blood. The Ficoll-Paque solution was warmed up to room temperature. 3L of bleach in 1:10 dilution was prepared, and used to wash all the tubes and pipettes contacting the blood. The blood was layered on the top of the Ficoll-Paque solution without disturbing the Ficoll layer, at 1.5ml blood/1ml of Ficoll-Paque. The tubes were centrifuged at 1700 rpm for 30 minutes at room temperature with the brake on the centrifuge turned off. As much of the top layer (plasma) as possible was removed, minimizing the vacuum in order to avoid removing the second layer of solution. The second layer, which contains the B and T lymphocytes, was collected using a sterile Pasteur pipette, and place in two 50 ml polystyrene tubes. The collection was diluted with 3x the volume of cold RPMI with no additives, and the tubes were centrifuged at 1000 RPM for 10 minutes. The media was removed by aspiratation, and the cells from both 50 ml tubes were resuspended in a total of 10ml cold RPMI (with additives) and transferred to a 15ml tube. The cells were counted using the hemacytometer, then centrifuged at 1000 RPM for 1 0 minutes. The media was removed and the cells resuspended in 4mls RPMI. This fraction contained the PBMC.

### Isolation of the B cells from PBMC.

100µl of Dynabeads (anti-hCD19) were placed in a 5 ml plastic tube.
3ml
of sterile PBS were added to the beads and mixed, and placed in the magnetic holder, then allowed to sit for 2 minutes. The solution was removed using a Pasteur pipette. 3mls of sterile PBS were added, mixed and placed in the magnetic holder, then let sit for 2 minutes. This procedure with sterile PBS was repeated one more time for a total of 3 washes. The PBMC was added into the beads and incubated, while mixing, for 30 minutes at 4°C. The tube containing the PBMC and beads was placed into the magnetic holder for 2 minutes, then the solution was transferred to a new 5ml tube in the magnetic holder. After 2 minutes, the solution was transferred to a new 15ml tube. This step was repeated four more times, and the solutions of the first four times were collected in the 15ml tube, then centrifuged at 1000 RPM for 5 minutes. This step produced the pellet for T cell separation.

100µl RPMI (with additives) was added to collect the beads, and the solution was transferred into a 0.7ml tube. 10µl of Dynal DetachaBeads were added into the suspension at room temperature, and it was allowed to rotate for 45 minutes. The suspension was transferred into a new 5ml tube and 3mls of RPMI (with additives) was added. The tube was placed in the magnetic holder for 2 minutes. The solution was transferred into a new 5ml tube in the holder for 2 minutes, then to a 15ml tube. The previous step was repeated three more times, collecting the solution in the 15ml tube. The 15ml tube was centrifuged at 1000RPM for 10 minutes, and the cells resuspended in 10ml RMPI. The washing step was repeated 2 more times for a total of 3 washes. The cells were counted before the last centrifugation. This step completed the B cell purification. Cells were stored in 90% FCS and 10% DMSO and frozen at - 80°C.

### Isolation of the T Cells.

The human T cell Enrichment Column (R&D systems, anti-hCD3 column kit) was prepared using 20ml of I X column wash buffer by mixing 2ml of 10X column wash buffer and 18ml of sterile distilled water. The column was cleaned with 70% ethanol and placed on top of a 15ml tube. The top cap of the column was removed first to avoid drawing air into the bottom of the column. Next, the bottom cap was removed, and the tip was cleaned with 70% ethanol. The fluid within the column was allowed to drain into the 15ml tube. A new sterile 15ml tube was placed under the column after the column buffer had drained to the level of the white filter. The B cell depleted PBMC fraction was suspended in 1ml of buffer and added it to the top of the column. The cells were allowed to incubate with the column at room temperature for 10 minutes. The T cells were eluted from the column with 4 aliquots of 2ml each of 1X column wash buffer. The collected T cells were centrifuged at 1000 RPM for 5 minutes. The supernatant was removed and the cells resuspended in 10mls RPMI. Cells were counted and centrifuged one more time. The supernatant was removed, completing the T cell purification. Cells were stored in 90% FCS and 10% DMSO and frozen at -80°C.

For the above procedures, the RPMI composition contained 10 % FCS (inactivated at 56°C for 45 min.), 1% Pen/Strep (100u/ml Penicillin, 0.1µg/ml Streptomycin), 1% Glutamate, 1% sodium puravate, 50µM 2-ME.

### Flow Cytofluorometric Assay

Ramos cells (10⁶ cells/sample) were incubated in 100 µl primary antibody (10 µg/ml in PBS-BSA) for 20 min at 4°C. After 3 washes with PBS-BSA or HBSS-BSA, the cells were incubated in 100 µl FITC-labeled F(ab')₂ fragments of goat anti-(human IgG) antibodies (Caltaq) for 20 min at 4°C. After 3 washes with PBS-BSA and 1 wash with PBS, the cells were resuspended in 0.5 ml PBS. Analyses were performed with a FACSCAN V (Becton Dickinson, San Jose, Calif.).

### Generation of Hybridoma Clones

Splenocytes from immunized mice were fused with SP2/0 or P3 x 63Ag8.653 murine myeloma cells at a ratio of 10:1 using 50% polyethylene glycol as previously described by de Boer et al., J Immunol. Meth. 113:143 (1988). The fused cells were resuspended in complete IMDM medium supplemented with hypoxanthine (0.1 mM), aminopterin (0.01 mM), thymidine (0.016 mM) and 0.5 ng/ml hIL-6 (Genzyme, Cambridge, Mass.). The fused cells were then distributed between the wells of 96-well tissue culture plates, so that each well contained 1 growing hybridoma on average.

After 10-14 days the supernatants of the hybridoma populations were screened for specific antibody production. For the screening of specific antibody production by the hybridoma clones, the supernatants from each well were pooled and tested for anti-CD40 activity specificity by ELISA first. The positives were then used for fluorescent cell staining of EBV-transformed B cells as described for the FACS Assay above. Positive hybridoma cells were cloned twice by limiting dilution in IMDM/FBS containing 0.5 ng/ml hIL-6.

### EXAMPLE 1

### EXPRESSION OF HUMAN CD40 IN SF9 CELLS

Sf9 insect cells infected with recombinant virus Autographa californica baculovirus (AcNPV), encoding CD40, were cultured for 48 hours in 24-well plates. After removal of the tissue culture medium the plates were incubated for 45 minutes at room temperature (RT) with 0.25 ml of antibody in PBS with 1% BSA (PBS-BSA). After three washes with PBS-BSA, the plates were incubated for 35 minutes at RT with 250 µl of a 1/250 dilution of goat anti-(mouse total Ig) immunoglobulins conjugated to horseradish peroxidase (Zymed, South San Francisco, Calif.) in PBS-BSA. Unbound peroxidase activity was removed by washing five times with PBS-BSA. Bound peroxidase activity was revealed by the addition of an assay mixture prepared by diluting 0.5 ml of 2 mg/ml 3,3',5,5'-tetramethylbenzidine in ethanol to 10 ml with 10 mM sodium acetate, 10 mM EDTA buffer (pH 5.0) and adding 0.03% (v/v) H.₂ O₂. The reaction was stopped after 10 minutes by adding 100 µl of 1 M H₂ SO₄.

### EXAMPLE 2

### ANTIBODIES RAISED IN HUMAN IMMUNOGLOBULIN TRANSGENIC MICE

Mice transgenic for the human IgG2 heavy chain locus and the human K light chain locus were immunized with Sf9 cells expressing human CD40. The method of immunization was carried out as described by de Boer, U.S. Patent No. 6,004,552. Briefly, mice were injected intraperitoneally at day 0 and day 14 with 5 x 10⁶ Sf9 cells infected with AcCD40 virus. At day 21, 100 µl of serum was obtained to test for the presence of specific antibodies. After a rest period of at least two weeks, the mice received a final injection with 5 x 10⁶ cells infected with AcCD40 virus. Three days after this last injection, the spleen cells were used for cell fusion.

Mice were selected for fusion based on the reactivity of their sera with recombinant CD40 in an ELISA. Spleen cells from immunized mice were fused with mouse myeloma cells (NS/0) by the method of Kohler and Milstein, Nature 256:495-96 (1975), with modifications. Hybridomas grown in HAT selective medium were selected for further characterization based on their ability to bind CD40 in an ELISA. Hybridomas that produced antibodies that bound nontransfected Sf9 cell lysate or anti-mouse light chain antibody were dropped from consideration. Hybridomas that produced CD40-binding antibodies that did not bind Sf9 cell lysate or anti-mouse light chain antibody were subcloned. Subclones were used to produce antibodies for further characterization. Hybridoma antibodies were also tested for their ability to stain Ramos lymphoma cells, which express human CD40 on their surfaces. The concentration of antibodies giving half-maximal CD40 binding as measured by ELISA is shown below in Table 2.

**Table 2 Concentration of anti-CD40 antibody giving half-maximal CD40 binding by ELISA**

| **Antibody** | **EC50 (ng/ml)** |
|---|---|
| 5H7 | 10 |
| 12D9 | 15 |
| 15B8 | 40 |
| 20C4 | 15 |
| 9F7 | 15 |

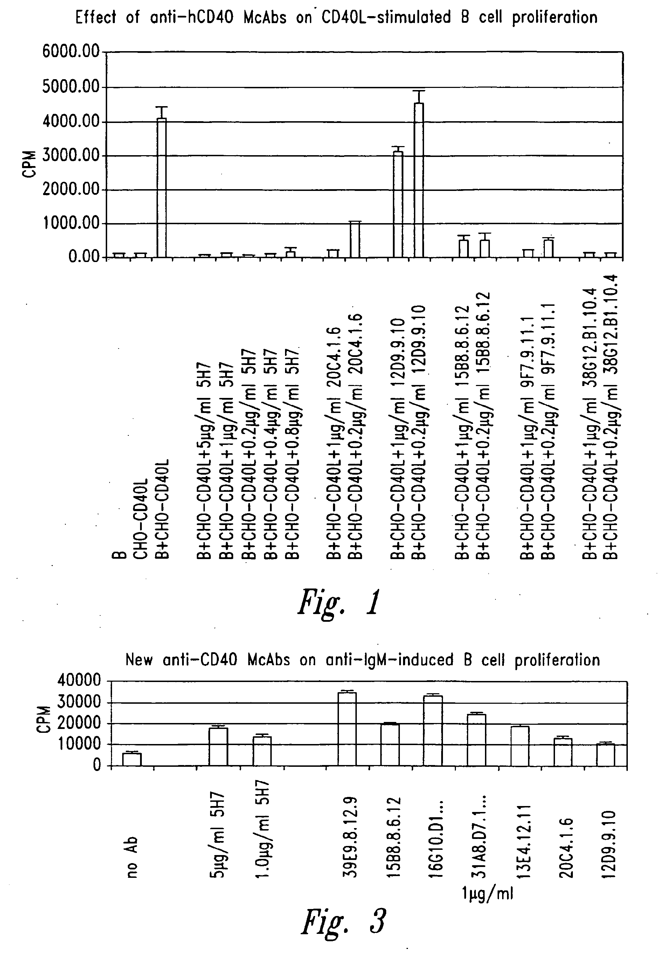

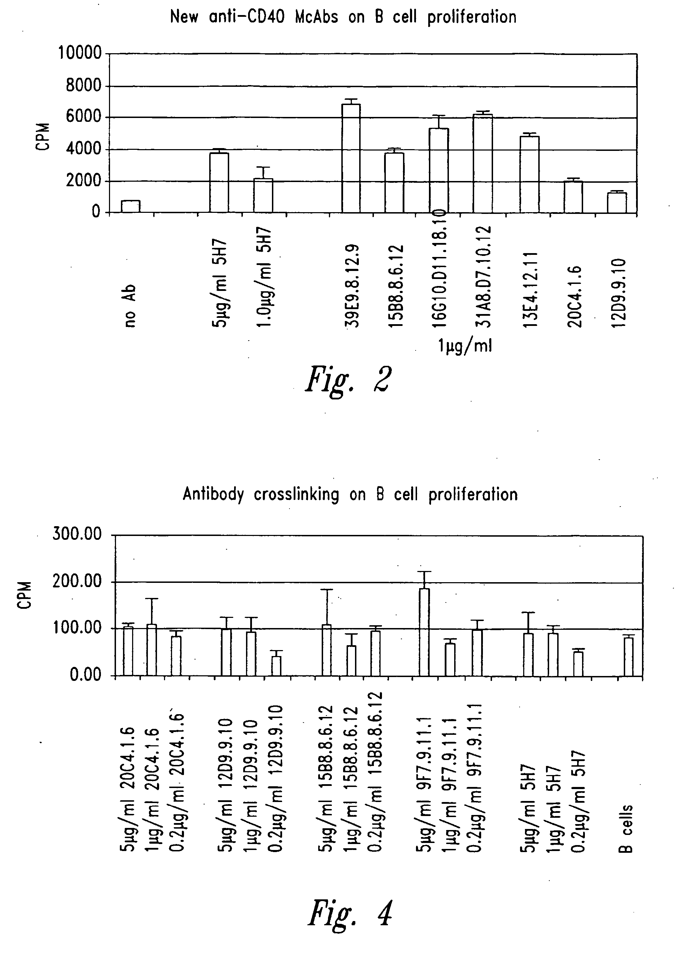

Hybridoma antibodies were then selected for their ability to inhibit the production of IgM by human peripheral blood B cells stimulated with anti-CD28-activated human peripheral blood T cells (Figure 1). Hybridoma antibodies were further screened for their ability to inhibit proliferation of human peripheral blood B cells induced by CD40L and anti-IgM (Figure 2). Hybridomas were also screened for their ability to induce proliferation in resting human peripheral blood B cells (Figure 3). Some hybridomas, such as 36C4-G2, exhibited marked stimulatory activity. Thus, even when able to bind CD40, not all human antibodies exhibit the desired inhibitory effect. Four hybridomas were selected from approximately 36 as having the optimal inhibitory activity.

### EXAMPLE 3

### BINDING PROPERTIES OF SELECTED HYBRIDOMAS

Four hybridomas were selected based on their inhibitory effect on B cell activation as described above in Example 2. Their binding properties were determined by BIAcore evaluation using soluble recombinant CD40 as the mobile phase with the anti-CD40 antibodies being captured on the sensor surface. The inhibitory antibodies exhibited various binding affinities, which are suitable for the uses described herein, as shown in Table 3.

**Table 3**

| **Antibody** | **Ka (M-1s-1)** | **Kd (sec⁻¹)** | **KD (M)** |
|---|---|---|---|
| 5H7 F_{ab}* | 2.4 x 10⁶ | 3.5 x 10⁻³ | 1.4 x 10⁻⁹ |
| 5H7 Ab** | 1.5 x 10⁶ | 4.1 x 10⁻³ | 2.8 x 10⁻⁹ |
| 15B8** | 1.16 x 10⁶ | 3.6 x 10⁻³ | 3.1 x 10⁻⁹ |
| 20C4** | 1.9 x 10⁵ | 3.6 x 10⁻² | 1.8 x 10⁻⁷ |
| 12D9** | 1.5 x 10⁵ | 1.0 x 10⁻¹ | 6.7 x 10⁻⁷ |
| 9F7** | 1.35 x 10⁵ | 2.2 x 10⁻² | 1.7 x 10⁻⁷ |

| | | | |
|---|---|---|---|
| *Fixed phase = CD40, Mobile phase = F_{ab} **Fixed phase = antibody, Mobile phase = sCD40 | | | |

### EXAMPLE 4

### VARIABLE HYBRIDOMA BINDING TO CD40

The relative binding properties of the selected monoclonal antibodies were examined by flow cytometry. Whole blood was incubated for 30 minutes with various concentrations of unlabeled hybridoma antibody plus 0.1 µg of FITC-conjugated 15B8 and anti-CD20-PECy5. The RBC's were then lysed, the leukocyte populations fixed, and the preparations acquired for analysis.

These studies show that unlabeled 15B8 blocks the binding of unlabeled 20C4 to CD20+ lymphocytes from human peripheral blood (Table 4). In contrast, unlabeled 20C4 only weakly blocked the binding of labeled 15B8 to the same cell population. Thus, although the two antibodies recognize the same or closely associated epitopes, the difference in affinity shown in the Biacore analysis also is reflected in the weaker competitive ability of the lower-affinity antibody. The labeled MAb's were used to stain peripheral blood cells from humans, Rhesus (*Macaca mulatta*), and cynomolgus (*Macaca fascicularis*) macaques. All three species were stained by the 15B8, 20C4, 12D9, and 9F7 MAb's (Figure 7).

**Table 4**

| **Tube #** | **Antibody-1** | µ**g** | **Antibody-2** | µ**g** | **huWB #1219** | **MFI*** | **% MFI reduction** |
|---|---|---|---|---|---|---|---|
| 1 | - | - | 15B6-FITC | 0.1 | 100 µl | 72.0 | |
| 2 | HulgG2 | 0.1 | 15B8-FITC | 0.1 | 100 µl | 117.5 | -63.19% |
| 3 | HulgG2 | 0.5 | 15B8-FITC | 0.1 | 100 µl | 97.0 | -34.72% |
| 4 | HulgG2 | 1 | 15B8-FITC | 0.1 | 100 µl | 103.8 | -44.17% |
| 5 | 15B8 | 0.1 | 15B8-FITC | 0.1 | 100 µl | 22.0 | 69.44% |
| 6 | 15B8 | 0.5 | 15B8-FITC | 0.1 | 100 µl | 20.5 | 71.53% |
| 7 | 15B8 | 1 | 15B8-FITC | 0.1 | 100 µl | 25.9 | 64.03 |
| 8 | 20C4 | 0.1 | 15B8-FITC | 0.1 | 100 µl | 91.6 | -27.22% |
| 9 | 20C4 | 0.5 | 15B8-FITC | 0.1 | 100 µl | 70.3 | 2.36% |
| 10 | 20C4 | 1 | 15B8-FITC | 0.1 | 100 µl | 61.0 | 15.28% |
| 11 | - | - | 20C4-FITC | 0.1 | 100 µl | 82.3 | |
| 12 | HulgG2 | 0.1 | 20C4-FITC | 0.1 | 100 µl | 82.6 | -0.61% |
| 13 | HulgG2 | 0.5 | 20C4-FITC | 0.1 | 100 µl | 74.4 | 9.60% |
| 14 | HulgG2 | 1 | 20C4-FITC | 0.1 | 100 µl | 82.4 | -0.12% |
| 15 | 15B8 | 0.1 | 20C4-FITC | 0.1 | 100 µl | 18.3 | 77.76% |
| 16 | 15B8 | 0.5 | 20C4-FITC | 0.1 | 100 µl | 21.3 | 74.12% |
| 17 | 15B8 | 1 | 20C4-FITC | 0.1 | 100 µl | 22.3 | 72.90% |
| 18 | 20C4 | 0.1 | 20C4-FITC | 0.1 | 100 µl | 30.2 | 63.30% |
| 19 | 20C4 | 0.5 | 20C4-FITC | 0.1 | 100 µl | 22.6 | 72.54% |
| 20 | 20C4 | 1 | 20C4-FITC | 0.1 | 100 µl | 20.8 | 74.73% |

| | | | | | | | |
|---|---|---|---|---|---|---|---|
| * MFI: mean fluorescent intensity | | | | | | | |

### EXAMPLE 5

### INHIBITION OF IMMUNOGLOBULIN SECRETION BY HUMAN PERIPHERAL B CELLS

Plates were coated with anti-human CD3 (2µg/ml, UCHTi, NE/LE, Pharmingen), at 4°C overnight. The pre-coated plates were washed 3 x with PBS. The T cells were irradiated with 3000 Rad. The B cells were resuspended in RPMI(+) to 10⁴ per ml. 100µl of B cells were added into the well, then anti-CD40 antibodies were added into the well. The T cells were resuspended in RPMI(+) to 10⁵ per ml. Human recombinant IL2 to 200u/ml was added (Chiron, 10u/µl water solution stored at -20°C) was added in the cell suspension. A 100µl suspension was taken into each well, and mixed well with the B cells and antibodies. RPMI(+) was added to the wells to a total of 200µl. The plates were incubated at 37°C for 8 days before harvesting the media, after spinning down the cells for ELISA. The results are shown below in Table 5.

**Table 5**

| Effect of monoclonal antibodies on IgM secretion by T cell-stimulated B cells | |
|---|---|
| **Antibody** | **O.D.** |
| None | 0.67 |
| 15B8 | 0.35 |
| 20C4 | 0.43 |
| 12D9 | 0.47 |
| 13E4 | 0.37 |

The results show that in the presence of an antibody according to the invention, the secretion of immunoglobulin, IgM, by T cell-stimulated human peripheral blood B cells, is decreased.

### EXAMPLE 6

### INHIBITION OF PROLIFERATION OF JURKAT-STIMULATED HUMAN PERIPHERAL B CELLS

B cells were purified as described above. 10⁴ purified B cells, 10⁵ irradiated Jurkat cells (3000 Rad), and antibodies to be tested were added into anti-CD3-coated 96-well plates. The plates were incubated at 37°C for four days, with labeling of the cells with ³H-thymidine during the last 18 hours. The cells were harvested and counted. The results are shown below in Table 6.

**Table 6**

| Effect of antibodies on Jurkat cell-stimulated B cell proliferation | |
|---|---|
| **Antibody** | **Counts per minute** **(cpm)** |
| None | 14,000 |
| 5H7, 10 µg/ml | 8,000 |
| 5H7, 0.1 µg/ml | 9,000 |
| 15B8, 1 µg/ml | 10,500 |
| 13E4, 1 µg/ml | 11,000 |
| 20C4, 1 µg/ml | 10,500 |

The results indicate that in the presence of the antibodies of the invention, B cell proliferation stimulated by Jurkat cells, is inhibited.

### EXAMPLE 7

### INHIBITION OF PROLIFERATION OF CD40L-STIMULATED HUMAN PERIPHERAL B CELLS

B cells were purified as described above. 10⁴ purified B cells, 2 x 10⁴ Formaldehyde-fixed CHO-CD40L cells, and antibodies to be tested were added into anti-CD3-coated 96-well plates. The plates were incubated at 37°C for four days, with labeling of the cells with ³H-thymidine during the last 18 hours. The cells were harvested and counted. The results are shown below in Figure 1.

### EXAMPLE 8

### SIMULATION OF B CELL PROLIFERATION

B cells (1 x 10⁴ per well) were cultured in 200 µl RPMI supplemented with 10% fetal calf serum in U-bottom 96-well microtiter plates. B cells were stimulated by addition of immobilized anti-(IgM) antibodies (5 µg/ml, Sigma). Varying concentrations of MAbs were added at the onset of the microcultures and proliferation was assessed at day 4 by measurement of the incorporation of ³H-thymidine after 18 hour pulsing. The results are shown in Figure 2.

### EXAMPLE 9

### EFFECT OF ANTI-CD40 ANTIBODIES ON PROLIFERATION OF HUMAN PERIPHERAL B CELLS STIMULATED BY ANTI-IGM

10⁴ purified B cells, anti-IgM beads (5 µg/ml, Sigma), and antibodies to be tested were added into 96 well plates. The plates were incubated at 37°C for four days, with labeling of the cells with ³H-thymidine during the last 18 hours. The cells were harvested and counted, and the results are shown in Figure 3.

### EXAMPLE 10

### EFFECT OF ANTI-CD40 ANTIBODY CROSSLINKING ON PROLIFERATION OF HUMAN PERIPHERAL B CELLS

10⁴ purified B cells were added into anti-CD40-coated 96 well plates. The plates were incubated at 37°C for four days, with labeling of the cells with ³H-thymidine during the last 18 hours. The cells were harvested and counted, and the results are shown in Figure 4.

### EXAMPLE 11

### POLYNUCLEOTIDE AND AMINO ACID SEQUENCES OF HUMAN ANTI-CD40 ANTIBODIES

mRNA was prepared from the hybridomas generated as described in Example 2 and RT-PCR was performed on the mRNAs. Two sets of primers were used to generate PCR products: a universal or pool of heavy and light chain family primers; and then family-specific primers. The PCR products were analyzed on gels, sequenced, and translated. The polynucleotide and amino acid sequences are provided in the Sequence Listing as summarized in Table 7.

**Table 7**

| **SEQ ID NO:** | **Antibody** | **Region** |
|---|---|---|
| 1 | 12D9 | Heavy chain constant region polynucleotide |
| 2 | 12D9 | Heavy chain constant region amino acids |
| 3 | 20C4 | VK1 polynucleotide |
| 4 | 20C4 | VH1 polynucleotide |
| 5 | 9F7 | VK1 polynucleotide |
| 6 | 9F7 | VH1 polynucleotide |
| 7 | 15B8 | VK3 polynucleotide |
| 8 | 15B8 | VH1 polynucleotide |
| 9 | 13E4 | VH1 polynucleotide |
| 10 | 12D9 | VH1 polynucleotide |
| 11 | 9F7 | VH1 amino acids |
| 12 | 12D9 | VH1 amino acids |
| 13 | 15B8 | VH1 amino acids |
| 14 | 20C4 | VH1 amino acids |
| 15 | 9F7 | VK1 amino acids |
| 16 | 12D9 | VK1 amino acids |
| 17 | 15B8 | VK1 amino acids |
| 18 | 20C4 | VK1 amino acids |

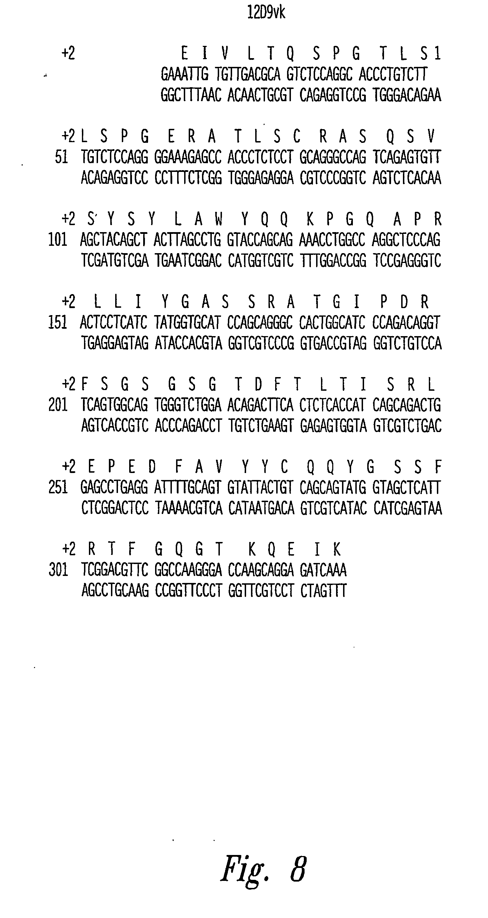

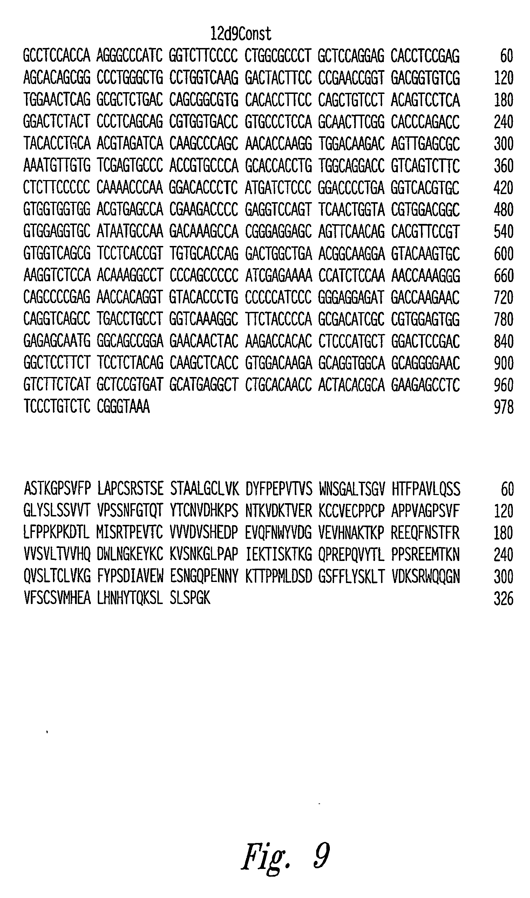

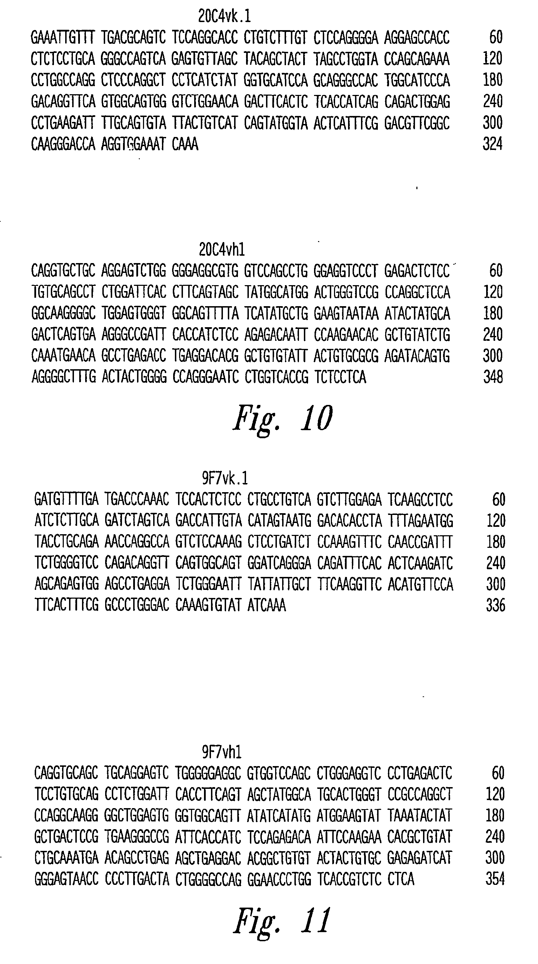

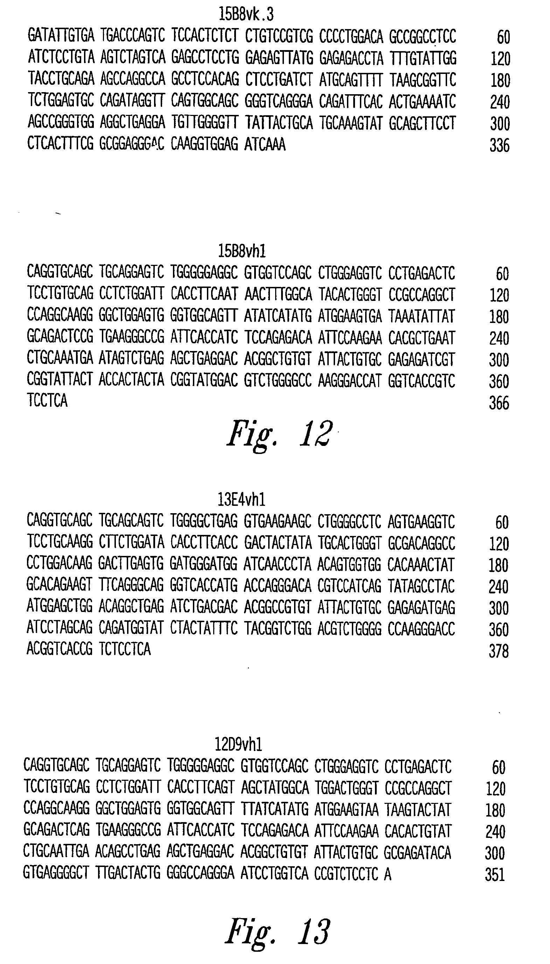

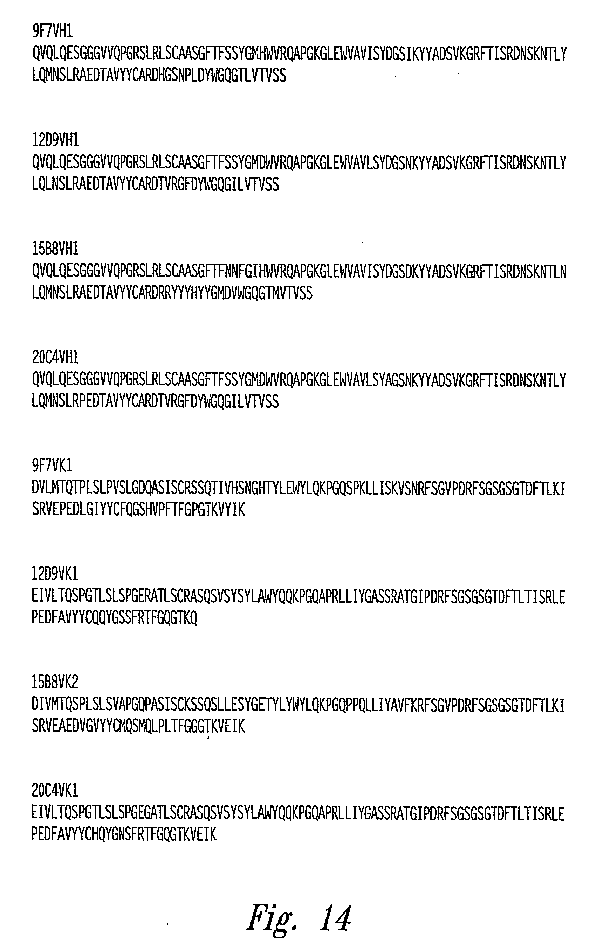

Specific regions of the antibodies are shown in Figures 5, 6, and 8-14. This information can be used to design additional monoclonal antibodies for use according to the invention. These monoclonal antibodies may differ from those described herein, by substitution of one or more of the framework or CDR regions. The monoclonal antibodies also may differ by substitution of one or more amino acids, which are shown to differ in certain regions of the framework and CDR (Figures 5 and 6). Once the amino acid sequence is designed, routine procedures can be used to construct a corresponding polynucleotide sequence for expression of the monoclonal antibody. Expression and purification of the monoclonal antibodies is performed using methods known in the art, such as those disclosed in U. S. Patent Nos. 5,545,403, 5,545,405, and 5,998,144, which are incorporated herein by reference.

### EXAMPLE 12

### EFFECT OF 15B8 ON MALIGNANT B-CELL PROLIFERATION IN IN VITRO

To test if 15B8 provides the growth signal like CD40L *in vitro,* B cells from tumor infiltrated lymph nodes (NHL cells) were obtained from one antibody naïve, one rituximab-sensitive and one rituximab-resistant NHL patient. The NHL cells were studied under four different culture conditions; no added antibody (medium); addition of human isotype antibody IgG2 (control); addition of anti-CD40 antibody MS81 (agonistic antibody); and addition of 15B8. All antibodies were tested 1, 2, and 5 µg/mL in the presence or absence of IL-4. The NHL cells from two patients were cultured as described above under the same four conditions in the presence of IL-4 (2 ng/ml). B-cell proliferation was measured by ³H-thymidine incorporation as described above.

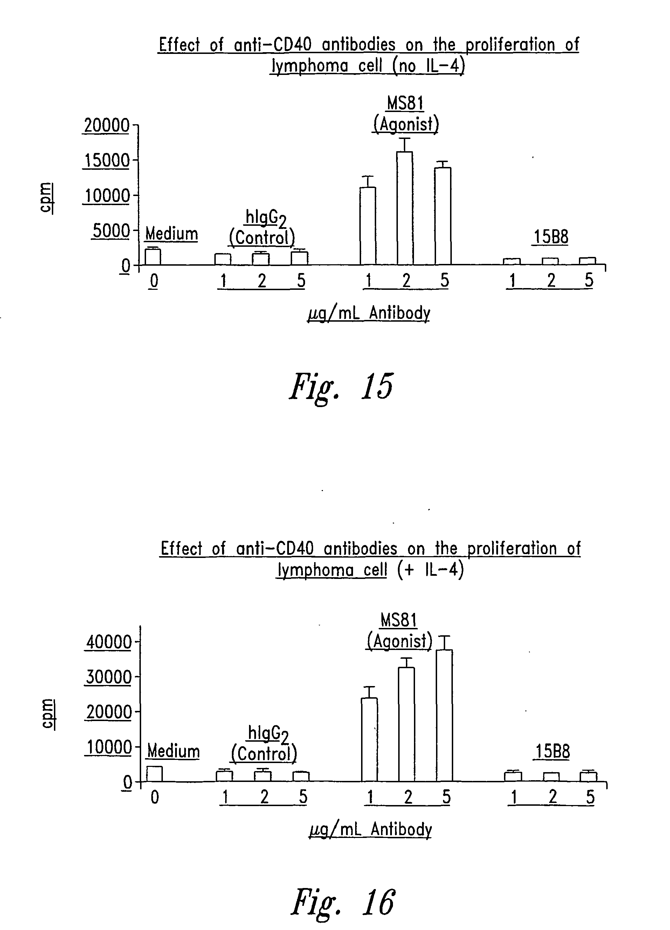

Anti-CD40 antibody 15B8, at concentration of 1, 2 and 5 µg/mL, did no stimulate NHL cells to proliferate in either the presence or absence of IL-4. In contrast, an agonistic anti-CD40 antibody (MS81), tested at the same concentration, stimulated NHL-cell proliferation or in the presence and absence of IL-4 in all patient samples. Representative results from one patient are shown below (Fig. 15 and Fig. 16). Results from the NHL cells from the two patients in the presence of IL-4 and three patients in the absence of IL-4 were comparable. These results indicate that 15B8 is not an agonist anti-CD40 antibody and does not stimulate proliferation of NHL cells from rituximab-sensitive, naïve or -resistant NHL patients *in vitro.*

FACS analysis of the NHL cells was performed with either a direct labelled 15B8-FITC or 15B8 plus anti-HuIgG2-FITC to confirm that CD40 is expressed on the surface the NHL cells tested and that 15B8 binds to the NHL cells. The NHL cells from two rituximab-sensitive and four rituximab-resistant patients (6 patients in total) were tested. NHL cells from all the patients expressed CD40 and bound to 15B8. The 15B8 binding-positive cell population in any given patient is about 66% to 91 %.

### EXAMPLE 13

### 15B8 INHIBITS CD40L-STIMULATED PROLIFERATION OF NHL CELLS IN VITRO

To evaluate the ability of 15B8 to block the growth signal provided by CD40L *in vitro,* NHL cells from patients were cultured as described in Example 12 in suspension over CD40L-expressing feeder cells under four different conditions: no added antibody (medium); addition of human isotype antibody IgG2 (control); addition of anti-CD40 antibody MS81 (agonistic antibody); and addition of 15B8. All antibodies were added at concentration of 1, 2, and 5 µg/mL in the presence or the absence of IL-4. The NHL cells from one antibody naïve, two rituximab-sensitive and five rituximab-resistant patients (8 patients in total) were cultured under the same four conditions as described above in the presence of IL-4 (2 ng/ml). NHL cells from three rituximab-sensitive and four rituximab-resistant patients (7 patients in total) were cultured under similar conditions in the absence of IL-4. The NHL cell proliferation was measured by ³H-thymidine incorporation.

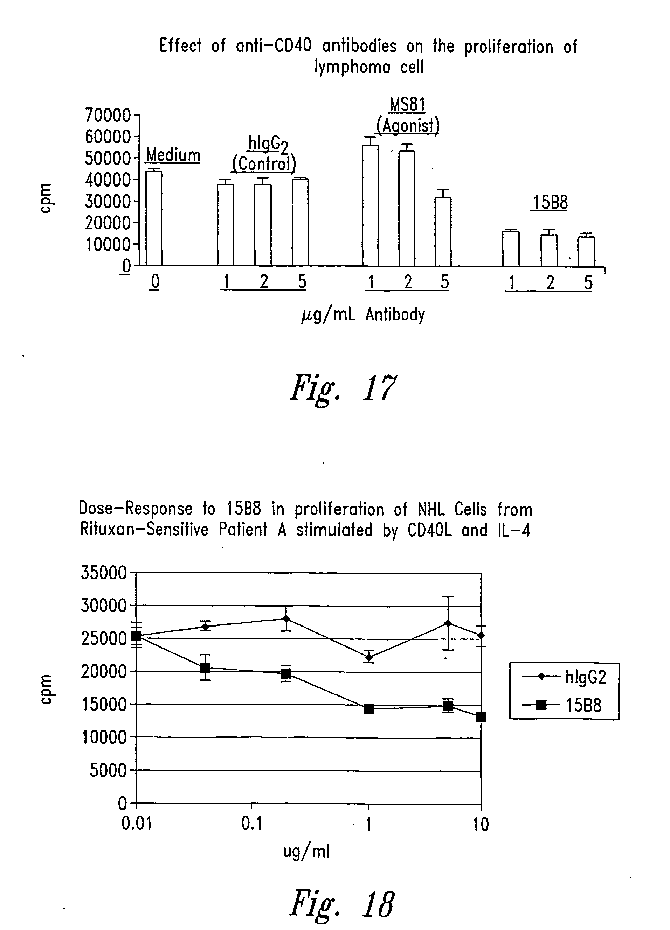

Table 8 below shows the inhibitory effect of 15B8 on the proliferation of NHL cells from two rituximab-sensitive (data from one patient reproducible in two separate experiments) and four rituximab-resistant patients (6 patients in total) stimulated by CD40L alone *in vitro.* Representative results from the cells of one patient (A) are shown (Fig. 17). 15B8 inhibited the proliferation by about 12-68% when compared to the control in the six patients. The degree of inhibition by 15B8 varies depending on patient samples and the dose level of 15B8. Statistical analysis of the data from six of the seven patient samples tested shows that the inhibition of CD40L-stimulated NHL cell proliferation by 15B8 is significant at I µg/ml (p=0.05). There is a statistically significant dose response (p<0.005), the inhibitory effect increases with increasing 15B8 dose.

**Table 8**

| Effect of 15B8 Mab on CD40-L Stimulated Proliferation of NHL Patient Cells in the Absence of IL-4¹ | | | |
|---|---|---|---|
| patient ID | patient type² | treatment dose (ug/ml) | 15B8 % inhib.³ |
| A | CR | 1 | 56.61 |
| | | 2 | 58.99 |
| | | 5 | 63.16 |
| A | CR | 1 | 61.96 |
| | | 2 | 60.41 |
| | | 5 | 64.75 |
| | | 10 | 60.29 |
| B | CR | 1 | none |
| | | 2 | none |
| | | 5 | none |
| | | 10 | 12.11 |
| D | NR | 1 | 52.22 |
| | | 2 | 61.63 |
| | | 5 | 68.04 |
| | | 10 | 68.17 |
| E | NR | 1 | 13.07 |
| | | 2 | 22.34 |
| | | 5 | 31.04 |
| | | 10 | 31.87 |
| F | NR | 1 | 24.51 |
| | | 2 | 27.43 |
| | | 5 | 38.71 |
| | | 10 | 47.35 |
| G | NR | 1 | 11.12 |
| | | 2 | 22.41 |
| | | 5 | 30.61 |
| | | 10 | 43.15 |

| | | | |
|---|---|---|---|
| 1. NHL cells from patients were cultured with murine L-cells expressing human CD40L in the presence of medium, agonist anti-CD40 (MS81), antagonist anti-CD40 (15B8) or huIgG2 isotype control *in vitro.* The proliferation of the NHL cells was measured by 3H-thymidine incorporation (data from one rituximab-sensitive patient is not in the table for the cpm of CD40L is<2000). 2. Patient response to anti-CD20 Mab therapy; CR, complete responder; NR, nonresponder 3. 15B8 % inhibition = 100 - (15B8 cpm/huIgG2 cpm x 100); represents the mean of 3 determinations | | | |

Table 9 (below) shows the inhibitory effect of 15B8 on proliferation of NHL cells from one antibody naïve, two rituximab-sensitive (data from both patient samples were repeated twice reproducibly) and five rituximab-resistant patients (8 patients in total) stimulated by both CD40L and IL-4 *in vitro.* At 1 µg/ml, 15B8 significantly (p<0.05) inhibited the CD40L and IL-4-mediated proliferation of the NHL cells. The degree of inhibition ranged from 18-69% at high dose (5 or 10 µg/ml) in samples from all 8 patients *in vitro.* There is a statistically significant dose response of this inhibitory effect by 15B8 (p<0.005) (Fig. 18 shows one representative dose response curve) at 15B8 concentration range of 0.01 - 10 µg/ml.

These *in vitro* results suggest that treatment with 15B8 may block the CD40-mediated growth signal for NHL cells in patients.

**Table 9**

| Effect on 15B8 MAb on CD40-L Stimulation of NHL Patient Cells in the Presence of IL-4¹ | | | |
|---|---|---|---|
| A | CR | 1 | 34.39 |
| | | 2 | 30.54 |
| | | 5 | 36.42 |
| A | CR | 0.01 | 0.44 |
| | | 0.04 | 23.32 |
| | | 0.2 | 29.54 |
| | | 1 | 35.38 |
| | | 5 | 46.12 |
| | | 10 | 48.63 |
| C | CR | 1 | 34.91 |
| | | 2 | 40.89 |
| | | 5 | 56.34 |
| | | 10 | 69.21 |
| C | CR | 1 | none |
| | | 2 | 16.79 |
| | | 5 | 21.64 |
| | | 10 | 12.63 |
| D | NR | 1 | 1.95 |
| | | 2 | 6.43 |
| | | 5 | 20.95 |
| | | 10 | 26.31 |
| E | NR | 1 | 1.91 |
| | | 2 | 2.74 |
| | | 5 | 28.36 |
| | | 10 | 28.26 |
| F | NR | 1 | none |
| | | 2 | 11.76 |
| | | 5 | 27.54 |
| | | 10 | 34.07 |
| G | NR | 1 | 39.38 |
| | | 2 | 32.74 |
| | | 5 | 36.48 |
| | | 10 | 37.78 |
| H | NR | 1 | none |
| | | 2 | none |
| | | 5 | 7.81 |
| | | 10 | 18.47 |
| I | Naïve | 0.01 | none |
| | | 0.04 | 13.16 |
| | | 0.2 | 15.64 |
| | | 1 | 16.20 |
| | | 5 | 21.53 |
| | | 10 | 24.51 |

| | | | |
|---|---|---|---|
| 1. NHL cells from patients were cultured with murine L-cells expressing human CD40L in the presence of IL-4 (human interleukin-4) at 2 ng/ml under conditions described in Table 1. 2. Patient response to anti-CD20 Mab therapy; CR, complete responder; NR, nonresponder; Naïve, untreated 3. % inhibition compared to hIgG2. 15B8 % inhibition = 100- (15B8 cpm/hulgG2 cpmx100) | | | |

### EXAMPLE 14

### DEMONSTRATION OF AGONISTIC AND ANATAGONISTIC ACTIVITY OF 15B8 IN DIFFERENT SPECIES IN VITRO

To determine if it is an agonist or antagonist anti-CD40, 15B8 was tested in several *in vitro* assays described below using cells from humans and five different primate species, including champanzee (chimp), marmoset, cynomologus mokey, rhesus monkey and baboon.

15B8 does not activate human peripheral blood B cell and does not cause PBMC proliferation *in vitro* in human, chimp and marmoset. Activation of CD40 on human B cells obtained from peripheral blood leads to proliferation of the B cells (van Kooten C., J. Leukoc. Biol., 2000 Jan., 67(1):2-17; Denton M.D., Pediatr. Transplant., 1998 Feb., 2(1):6-15; Evans D.E., J. Immunol., 2000 Jan. 15, 164(2):688-97; Noelle R.J., Agents Actions Suppl., 1998, 49:17-22; Lederman S. et al., Curr. Opin. Hematol., 1996, 3(1):77-86). To test if 15B8 activates CD40 on B cells, a series of proliferation assays was carried out using freshly isolated human B cells or PBMCs from peripheral blood. The effect of 15B8 in this assay was measured by ³H methyl-thymidine incorporation (John E. Coligan et al., Current Protocols in Immunology, Vol. 13:12, John Wiley & Sons, Inc., 1991; Kwekkeboom J., Immunology, 1993 Jul, 79(3):439-444). As shown in Table 10 below at concentrations of 0.2, 1 and 5 µg/ml, 15B8 had minimal effect on purified B cell proliferation compared to the effect on CD40L, which demonstrated strong proliferation-promoting effect on human B cells. As shown in table 3, the results in purified B cells from two healthy volunteers are comparable.

15B8 was further compared to CD40L for stimulation of human PBMC proliferation using freshly isolated human PBMC. As summarized in Table 10 below, 15B8 does not stimulate human PBMC proliferation *in vitro* as measured by 3-H methyl-thymidine incorporation (John E. Coligan et al., Current Protocols in Immunology, Vol. 13:12, John Wiley & Sons, Inc., 1991; Kwekkeboom J., Immunology, 1993 Jul, 79(3):439-444) in samples tested from sixteen volunteers at concentration range of 0.2-5µg/ml.

**Table 10**

| Stimulation of PBMC/B cell-proliferation in human, chimp and marmoset by 15B8 antibody¹ | | | | | | |
|---|---|---|---|---|---|---|
| **Species** | **cell source** | **Number of samples** | **dose (ug/ml)** | **hlgG2, base** | **CD40L, fold increase³** | **15B8, fold increase²** |
| human | B | 2 | 5 | 1 | 70.58/36.33 | 1.77/4.37 |
| | | 2 | 1 | 1 | 70.58/36.33 | 3.1/5.4 |
| | | 2 | 0.2 | 1 | 70.58/36.33 | 1.16/4.63 |
| human | PBMC | 5 | 5 | 1 | 9.36-91.60 | 0.40-2.28 |
| | | 15 | 1 | 1 | 9.36-91.60 | 0.35-2.38 |
| | | 12 | 0.2 | 1 | 9.36-91.60 | 0.41-3.74 |
| Marmoset | | | | | | |
| Monkey | PBMC | 3 | 5 | 1 | 29.24-90.3 | 2.05-7.2 |
| | | 5 | 1 | 1 | 7.99-90.3 | 1.35-5.79 |
| Chimp PBMC | PBMC | 1 | 5 | 1 | 10.15 | 2.46 |
| | | 5 | 1 | 1 | 5.12-9.2 | 0.66-5.2 |

| | | | | | | |
|---|---|---|---|---|---|---|
| 1. B cells/PBMCs were cultured in vitro in the presence of CD40L, 15B8 or hulgG2 isotype control. 2. Results of the cell proliferation are reported as the ration of 3H-thymidine incorporation for 15B8 to huIG2 controls. Data from some samples are not included in the table for the CPM induced by CD40L (positive control) <2000. 3. The fold-increase for CD40L shown in the table is the ratio of the CD40L cpm to the cpm of huIG2 at 5µg/ml. CD40L: CD40L transfected CHO cells, fixed with formaldehyde before the experiments. | | | | | | |

Upon B cell activation, a number of cell surface proteins are up-regulated (Denton M.D., Pediatr. Transplant., 1998 Feb., 2(1):6-15; Evans D.E., J. Immunol., 2000 Jan. 15, 164(2):688-97; Noelle R.J., Agents Actions Suppl., 1998, 49:17-22; Lederman S. et al., Curr. Opin. Hematol., 1996, 3(1):77-86). To confirm that 15B8 does not activate human B cells and does not induce an agonist signal when bound to CD40, its ability to up-regulate B cell activation markers was tested by FACS analysis using purified human PBMC. There was no up-regulation in the expression of activation markers such as CD25, CD69, CD86, HLA-DR and ICAM-1 (CD54) in 15B8 treated human B cells (Table 4). The level of these markers was similar when cells were treated with either 15B8 or huIgG2 control (Table 11). In contrast, CD69 was consistently up-regulated by CD40L in PBMC samples from three healthy volunteers tested.

**Table 11**

| Effect of 15B8 on upregulation of B-cell activation markers in vitro by FACS | | | | | | | | | |
|---|---|---|---|---|---|---|---|---|---|
| **Species** | **cell source** | **incubation time** | **Number of subject** | **CD54** | **CD69** | **HLA- DR** | **CD25** | **CD80** | **CD86** |
| human | CD20 from PBMC | 4h-24h | 3 | - | - | - | - | N/A | - |
| Chimp | CD20 from PBMC | 4h-24h | 3 | N/A | - | N/A | N/A | N/A | N/A |

| | | | | | | | | | |
|---|---|---|---|---|---|---|---|---|---|
| 1 "-" means no up-regulation. 2 "N/A": means not measured or not successful. | | | | | | | | | |

Additional consequences of B cell activation are up-regulation of surface FasL and apoptosis (Revy P., Eur. J Immunol., 1998 Nov., 28(11):3648-3654; Carey G.B., Immunol. Rev., 2000 Aug., 176:105-115; Ju S.T., Int. Rev. Immunol., 1999, 18(5-6):485-513; Baumgarth N., Immunol. Rev., 2000 Aug., 176:171-180). To confirm that 15B8 is not an agonistic anti-CD40 antibody, its ability to induce FasL expression and apoptosis of human B cells was also tested. Annexin V staining on the cell surface can be used as an early apoptosis marker (Ju S.T., Int. Rev. Immunol., 1999, 18(5-6):485-513). Human B cells were purified from peripheral blood and incubated with 15B8. FACS analysis was used to detect cells with positive staining of Annexin V and anti-FasL. There was no significant difference on the surface staining by the two reagents between cells incubated with 15B8 or the isotype control (huIgG2) antibody. This result shows that 15B8 does not induce apoptosis of human B cells *in vitro.* These data provide further evidence that 15B8 is not an agonistic anti-CD40 antibody for human B cells.

15B8 cross-reacts with CD40 expressed on the surface of CD20 positive PBMCs from primates. To test if 15B8 can activate CD40 on B cells from other primate species such as chimps and marmosets, the same proliferation assay were carried out using freshly isolated chimp and marmoset PBMC from fifteen chimps and five marmosets. Similar to the results with the human PBMC, 15B8 did not stimulate the proliferation *in vitro* of PBMCs from six chimps and five marmosets at 1 and 5 µg/ml concentration (Table 3 above). 15B8 also did not up-regulate the expression of activation marker, CD69, in the three chimp-PBMC samples tested (Table 4). 15B8 did

not show any effect on FasL expression and apoptosis in chimp PBMCs similar to human PBMC controls after 24 and 48 hours simulation in vitro in all samples from six chimps tested.

Cross-linking 15B8 by a secondary antibody fixed to plastic surface did not increase its potency to stimulate B cell proliferation (data not shown). When tested using PBMCs from humans and chimps in this cross-linking assay, 15B8 did not stimulate proliferation of the cells. This observation indicates a reduced risk of 15B8 being stimulative (agonistic) for B cell proliferation in case of induction of anti-15B8 (HAHA) or Fc binding to other Fc receptor expressing cells when administered *in vivo.*

In summary, 15B8 does not initiate an activation signal in human B cells/PBMCs nor in chimp/marmoset PBMCs *in vitro.* Therefore, 15B8 is not an agonistic anti-CD40 antibody in humans, chimps and marmosets.

### EXAMPLE 15

### 15B8 IS AN ANTAGONIST ANTI-CD40 ANTIBODY IN HUMANS, CHIMPS AND MARMOSETS IN VITRO.

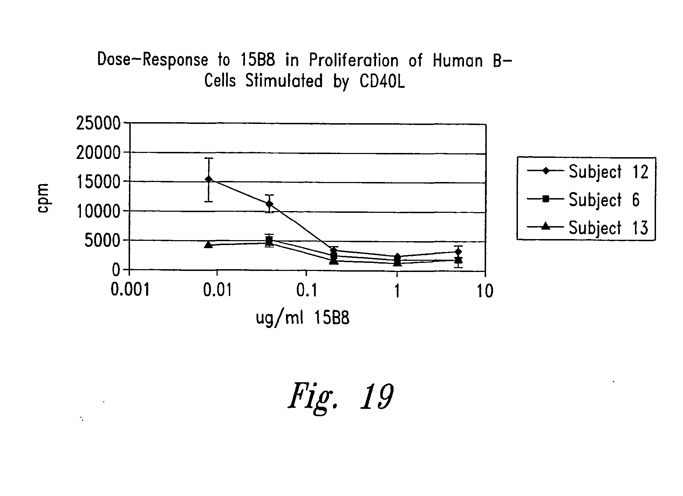

To determine if 15B8 is an antagonist anti-CD40, its ability to inhibit CD40-CD40L interaction was tested in a CD40L mediated-human B cell proliferation assay (Kwekkeboom J., Immunology, 1993 Jul, 79(3):439-444). A transfected CHO cell line expressing human CD40L was used to stimulate the proliferation of purified human peripheral blood B cells or PBMCs. Human B cells from ten healthy volunteers and human PBMCs from three healthy volunteers were tested. In all the samples tested, 15B8 suppressed CD40L-expressing CHO cells mediated-proliferation by 42-88% at concentration range from 0.2 - 5µg/ml (Table 12). Figure 19 shows representative dose-response curves using cells from three individuals. The no-effect dose of 15B8 is 0.008 µg/ml and reaches saturating dose at 0.2 µg/ml (Figure 19). This observation indicates that 15B8, as an antagonist anti-CD40 antibody, can inhibit the growth signals in human B cells and PBMCs provided by cell surface-expressed CD40L.

**Table 12**

| Inhibition of CD40L induced-proliferation of PBMCB cell with 15B8 antibody¹ | | | | | | |
|---|---|---|---|---|---|---|
| **Species** | **cell source** | **Number of samples** | **dose (ug/ml)** | **CD40L** **(base)** | **hIgG2, % of inhibition** | **15B8, fold inhibition2** |
| human | B | 7 | 5 | 100 | (-27) - 14% | 45-85% |
| | | 9 | 1 | 100 | (-93) -11% | 42-87% |
| | | 6 | 0.2 | 100 | (-20) - (-6)% | 44-82% |
| human | PBMC | 1 | 5 | 100 | 13% | 45% |
| | | 2 | 1 | 100 | 3-32% | 76-88% |
| Marmoset Monkey | PBMC | 3 | 1 | 100 | 1-35% | 68-84% |
| Chimp PBMC | PBMC | 3 | 1 | 100 | (-3) - 21% | 55-73% |

| | | | | | | |
|---|---|---|---|---|---|---|
| 1. B cells/PBMCs were cultured *in vitro* with CD40L expressing CHO cells in the presence of 15B8 or huIgG2 control. CD40L transfected CHO cells were fixed with formaldehyde before the experiments. The proliferation of cells was measured by 3H-thymidine incorporation. 2. "15B8 % inhibition" = 100- (15B8 cpm/CD40L cpm x 100) Data from some sample are not in the table for proliferation induced by CD40L (positive control) is -folds. | | | | | | |

Additional assays were carried out using freshly isolated PBMCs from nine chimps and three marmosets. As with the human PBMCs, 15B8 was able to inhibit the proliferation of chimp and marmoset PBMCs stimulated by CD40L expressing-CHO cells at 1µg/ml concentration level (Table 5 above). The inhibition by 15B8 was approximately 55-73% and 68-84% in PBMC samples from three chimps and three marmosets respectively (Table 5 above).

Activated B cells undergo a number of biological response such as proliferation and antibody production. The activation of B cells by T cell-dependent antigens involves CD4⁺ T-helper (Th) cells. This T cell helper process is mediated by a concerted effort of the interaction of CD40 on the B cells with the CD40L on the Th cells surface together with the interactions of other co-stimulatory factors and cytokinesDenton M.D., Pediatr. Transplant., 1998 Feb., 2(1):6-15; Evans D.E., J. Immunol., 2000 Jan. 15, 164(2):688-97; Noelle R.J., Agents Actions Suppl., 1998, 49:17-22; Lederman S. et al., Curr. Opin. Hematol., 1996, 3(1):77-86; Mackey M.F., et al., J. Leukoc. Biol., 1998, 63(4):418-428). To test if 15B8 can block T-helper cell mediated B cell antibody production, purified human peripheral blood B cells were cultured in the presence of purified irradiate T cells activated with anti-CD3 antibody. An ELISA assay was used to measure the level of IgM production. 15B8 reduced IgM production by about 30% in this assay (data not shown). Therefore, 15B8 can reduce T cell-mediated B cell immunoglobulin production.

In summary, 15B8 inhibits CD40L induced Bpcell/PBMC proliferation in human, chimp and marmoset, and inhibits T cell inducted antibody production by purified human B cells *in vitro.* These data demonstrate that 15B8 is an antagonistic anti-CD40 antibody in human B cells and PBMCs from chimps and marmosets *in vitro.*

### EXAMPLE 16

### 15B8 IS AN AGONIST ANTI-MONKEY (CYNOMOLGUS, RHESUS AND BABOON) CD40 ANTIBODY IN VITRO.

FACS analysis demonstrates that 15B8 binds to CD40 expressed on the surface of B cells from peripheral blood of monkeys (rhesus, cynomologus and baboon). The effect of 15B8 on freshly isolated cynomolgus monkey PBMC was tested in the same proliferation assay described above for human and chimps (John E. Coligan et al., Current Protocols in Immunology, Vol. 13:12, John Wiley & Sons, Inc., 1991; Kwekkeboom J., Immunology, 1993 Jul, 79(3):439-444). In contrast to human PBMC, 15B8 was found to stimulate cynomolgus monkey PBMC to proliferate *in vitro* as measured by ³H methyl-thymidine incorporation (Table 13 below). At 1 µg/ml level, 15B8 stimulated the proliferation of the PBMCs by 6 - 129.7 folds compare to the huIgG2 control in the tewnety-two samples from seventeen monkeys tested (samples from five monekys were tested twice) (Table 13 below). At 5µg/ml level, the proliferation stimulated by 15B8 is 14 - 24 folds in four samples from to monkeys and about 1.25 or 1.85 fold in two samples from two monkeys (Table 13). This suggests that, at concentration level of 5µg/ml, 15B8 may be at the limit of over-saturating dose for its proliferation-stimulatory effect on PBMCs from cynomolgus monkey. Further FACS analysis of B cells for activation status by surface markers indicated that 15B8 induces CD69, CD86 and HLA-DR up-regulation on monkey B cells (Table 14). These data suggest that 15B8 is an agonist antibody to CD40 expressed on peripheral blood B cells from cynomolgus monkeys *in vitro.*

To confirm that this agonistic effect of 15B8 is not cynomolgus monkey specific, the same assays were performed using PBMCs from rhesus monkeys and baboons. Similar results to that obtained from cells of cynomolgus monkeys were observed as shown in the Table 13 below. 15B8 stimulated proliferation of PBMCs from rhesus monkeys and baboons *in vitro* (Table 13 below). The agonist activity of 15B8 is shown using the PBMCs from five rhesus monkey and three baboons (Table 13).

**Table 13**

| Proliferation of PBMCs from humans, cynomolgus and rhesus monkeys, and baboons stimulated at 15B8¹ | | | | | | |
|---|---|---|---|---|---|---|
| **Species** | **cell source** | **Number of samples** | **dose (ug/ml)** | **hIgG2, base** | **CD40L, fold increase³** | **15B8, fold increase²** |
| human | PBMC | 5 | 5 | 1 | 9.36-91.60 | 0.49-2.28 |
| | | 15 | 1 | 1 | 9.36-91.60 | 0.35-2.38 |
| | | 12 | 0.2 | 1 | 9.36-91.60 | 0.41-3.74 |
| Rhesus Monkey | PBMC | 5 | 1 | 1 | 12.71-89.67 | 27.34-50.9 |
| cyno monkey | PBMC | 6 | 5 | 1 | 14.57-124.01 | 1.25-24.53 |
| | | 22 | 1 | 1 | 5.15-167.73 | 6.13-129.74 |
| | | 3 | 0.2 | 1 | 77.01-124.01 | 0.9-67.56 |
| Baboon | PBMC | 3 | 1 | 1 | 5.19-175.07 | 3.32-113.28 |

| | | | | | | |
|---|---|---|---|---|---|---|
| 1, PBMCs were cultured in vitro in the presence of CD40L, 15B8 or huIgG2 control. 2. The proliferation results are reported as the ration of ³H-thymidine incorporation for 15B8 to huIgG2 control. Data from some samples are not in the table for the CPM induced by CD40L (positive control) <2000. 3. The fold-increase for CD40L shown in the table is the ratio of the CD40L cpm to the cpm of huIgG2 at 5µg/ml. CD40L transfected CHO cells were fixed with formaldehyde before the experiments. | | | | | | |

**Table 14**

| Effect of 15B8 on upregulation of B-cell activate markers *in vitro* by FACS analysis | | | | | | | | | |
|---|---|---|---|---|---|---|---|---|---|
| **Species** | **cell source** | **incubation time** | **Number of subject** | **CD54** | **CD69** | **HLA-DR** | **CD25** | **CD80** | **CD86** |
| human | CD20 from PBMC | 4 h-24 h | 3 | - | - | - | - | N/A | - |
| cyno monkey | CD20/19 from PBMC | 4h-3 day | 2 | N/A | 1/2 up | 1/1 up (day 3) | - | - | 1/1/up (day 3) |

| | | | | | | | | | |
|---|---|---|---|---|---|---|---|---|---|
| 1, "-" means no up-regulation. 2, "N/A" means not measured or not successful. 3, Only cells from one cyno, analyzed by FACS on day 3. | | | | | | | | | |

### EXAMPLE 17

### 15B8 IS AN AGONIST ANTI CD40 ANTIBODY IN VIVO IN CYNOMOLGUS MONKEYS..

15B8 can stimulate proliferation and up-regulation of cell surface activation markers in PBMCs from cynomolgus monkeys *in vitro.* To determine if 15B8 is an agonist anti-CD40 antibody in the monkeys *in vivo,* a study was performed to examine the biodistribution of 15B8 and the fate of affected peripheral B cells (i.e. extravastion, apoptosis, activation status, or complement lysis) [Biodistribution 15B8.72 Antibodies following Intravenous Administration to Non-Naïve Male and Female Cynomolgus Monkeys (SNBL.218.3, SNBL USA)].

Cynomolgus monkeys (1 female and 2 males) received a single intravenous administration of 3 mg/kg 15B8. The following parameters were monitored: clinical signs, food consumption, body weight, pharmacokinetics, serum complement (CH50), flow cytometry for B cells (including apoptotic B cells), T cells, and monocytes. B cell CD40 receptor saturation with 15B8 was also measured. Animals were necropsied 24 hours after receiving the single dose of 15B8, and standard organs were weighed. Pre-study surgical biopsies of spleen and axiliary lymph nodes were taken to serve as baseline controls. At necropsy, lymphoid and non-lymphoid tissues were sampled for histopathology and immunohistochemistry. Tissues were immunostained with antibodies against CD3, CD40, CD20, CD27, and CD38 antigens. Preliminary results of the study are discussed below.

All animals survived to the scheduled necropsy and there were no effects on food consumption, body weight, CH50 levels nor on peripheral blood T cell or monocyte counts. There were no changes in organ weights. Microscopic examination of the spleen showed moderate diffuse follicular hyperplasia with necrosis and/or neutrophilic infiltrates in the germinal centers of all 15B8-treated animals. Examination of mesenteric and inguinal lymph nodes revealed mild follicular hyperplasia in 2/3 animals. No treatment related microscopic effects were seen in other tissues (liver, skin, brain, thyroid, lung, bone marrow, adrenal gland and kidney).

Immunostaining with CD20, CD27, CD40 and CD86 antibodies revealed increases in these markers in splenic and lymph node follicles, which correlated with the follicular hyperplasia seen in these same tissues. Increased staining of CD20 and CD40 were limited to the spleen and lymph node while there was some additional staining of hepatic tissue with CD27 and of hepatic Kupffer cells and inflammatory cells by CD86. CD86 staining was also increased in thymic medullary cells and adrenal interstitial leukocytes. There were no changes in the immunostaining of CD3 in 15B8-treated animals as compared to controls.

These findings indicate that a single dose of 3 mg/kg of 15B8 administered to cynomolgus monkey can cause proliferation of lymphoid follicles and/or redistribution of B cells from the peripheral blood in spleen and lymph nodes within a 24 hour period. Antibodies to CD20, CD27, CD40 and CD86 recognize antigens expressed on B cells and/or activated B cells, along with recognition of other cell types. Increased numbers of cells expressing these antigens were seen in the spleen and lymph nodes of treated animals, which suggests an increase in the number of activated CD20+ B cells. This study suggests that 15B8 is an agonist anti-CD40 antibody in cynomolgus monkey *in vivo.* The results obtained *in vivo* and *in vivo* are consistent in cynomolgus monkeys.

The results suggest that 15B8 is an agonistic anti-CD40 antibody in cynomolgus and rhesus monkeys and baboons, and an antagonistic antibody in humans, chimpanzees and marmosets.

15B8 is an anti-human CD40 specific monoclonal antibody with human IgG2 subtype and with cross-reactivity to CD40 from non-human primates only. Through extensive *in vitro* testing, 15B8 was shown to be an antagonistic anti-CD40 to the CD40 expressed on human B cells, PBMCs from human, chimp and marmoset. However, 15B8 was shown to have agonistic activity when bound to the CD40 expressed on PBMCs from monkeys (cynomolgus, rhesus and baboon) *in vitro.* This agonistic activity of 15B8 was confirmed *in vivo* in cynomolgus monkeys. When tested in primary culture of lymphoma cells from Rituxan-sensitive and resistant NHL patients, 15B8 has no agonist activity in the presence and absence of IL-4. 15B8 can also inhibit CD40L stimulated growth of the lymphoma cells from the similar group of patients under both conditions. 15B8 will have the potential to modify B cell malignancies, such as non-Hodgkin's lymphoma (NHL), where the CD40/CD40L pathway may play a role in the pathogenesis of the diseases. The results are summarized in the tables below.

**Table 15**

| Assays Measuring Agonistic Activity | | |
|---|---|---|
| **Assay** | **Methodology** | **Species Tested (+ or - Agonistic Activity)** |
| **Effect** of 15B8 on B cell proliferation | Compared ³H-thymidine incorporation of purified B cells from the peripheral blood in presence of 15B8 with incorporation in presence of CD40L or an agonistic antibody 626.1 | • Human (-) |
| Effect of 15B8 on PBMC proliferation | Compared ³H-thymidine incorporation of PBMCs in presence of 15B8 with incorporation in presence of CD40L or the isotype control | • Human (-) • Chimpanzee (-) • Cynomolgus monkey (+) • Rhesus monkey (+) • Baboon (+) • Marmoset (-) |
| Effect of 15B8 on upregulation of B-cell activation markers | Measured upregulation in the expression of B-cell activation markers in PBMCs stimulated by 15B8 or it's isotype control using FACS analysis; compared effect of 15B8 with that of isotype control | • Human (-) • Chimpanzee (-) • Cynomolgus monkey (+) • Rhesus monkey (+) • Baboon (+) • Marmoset (-) |
| Effect on PBMC proliferation of 15B8 cross-linked to a secondary antibody fixed to a plastic surface | Compared ³H-thymidine incorporation in presence of second Ab-crosslinked 15B8 with incorporation in presence of CD40L 15B8 alone or the isotype control | • Human (-) • Chimpanzee (-) |
| Effect of 15B8 on upregulation of FasL and apoptosis | Measured upregulation in the expression of FasL and apoptosis by FACS detection of B cells with positive staining of anti-FasL and Annexin V (marker for apoptosis) by the stimulation of CD40L, 15B8 and the isotype control. | • Human (-) • Chimps (-) • Cynomologus Monkey (-/+) |

**Table 16**

| Assays Measuring Antagonistic Activity | | |
|---|---|---|
| **Assay** | **Methodology** | **Species Tested (+ or - Antagonistic Activity** |
| Inhibition by 15B8 of CD40L-mediated B-cell proliferation | Stimulation of B-cel) proliferation by CD40L-expressing CHO cells was measured by ³H-thymidine incorporation. Compared H-thymidine incorporation in presence of 15B8 with that in presence of isoitype control | • Human (+) • Marmoset (+) • Chimps (+) |
| Inhibition by 15138 of T-helper-cell-mediated B-cell antibody production | B cells were cultured with purified irradiated T cells activated with anti-CD3 antibody in the presence of 15B8. The level of B-cell IgM production was assessed by ELISA. | • Human (+) |

## Claims

1. A human monoclonal antibody or fragment thereof that is capable of specifically binding to a human CD40 antigen expressed on the surface of a normal human B cell, said monoclonal antibody or fragment being free of significant agonistic activity when said monoclonal antibody or fragment binds to the CD40 antigen expressed on the surface of said normal B cell, whereby, when said monoclonal antibody or fragment binds to the CD40 antigen expressed on the surface of said normal B cell, the growth or differentiation of said normal B cell is inhibited, wherein said monoclonal antibody or fragment thereof comprises a heavy chain variable region containing residues 31-35, 50-66, and 99-111 of SEQ ID NO:13, and comprises a light chain variable region containing residues 24-39, 55-61, and 94-102 of SEQ ID NO:17.

2. The human monoclonal antibody or fragment thereof according to claim 1, which comprises the amino acid sequence set forth in SEQ ID NO:13.

3. The human monoclonal antibody or fragment thereof according to claim 1, which comprises the amino acid sequence set forth in SEQ ID NO:17.

4. The human monoclonal antibody or fragment thereof according to claim 1, which comprises the amino acid sequence set forth in SEQ ID NO:13 and the amino acid sequence set forth in SEQ ID NO:17.

5. The human monoclonal antibody or fragment thereof according to any preceding claim, which possesses at least one of the following biological activities:
(i) inhibition of immunoglobulin secretion by human peripheral B cells stimulated by T cells;
(ii) inhibition of proliferation of human peripheral B cells stimulated by Jurkat T cells; and
(iii) inhibition of proliferation of human peripheral B cells stimulated by CD40L-expressing cells.

6. The human monoclonal antibody or fragment thereof according to any preceding claim, which possesses the following biological activities:
(i) inhibition of immunoglobulin secretion by human peripheral B cells stimulated by T cells;
(ii) inhibition of proliferation of human peripheral B cells stimulated by Jurkat T cells; and
(iii) inhibition of proliferation of human peripheral B cells stimulated by CD40L-expressing cells.

7. The human monoclonal antibody or fragment thereof according to any preceding claim, wherein said antibody or fragment thereof binds to said human CD40 antigen with an affinity (K_{D}) selected from the group consisting of at least 10⁻⁵ M, at least 10⁻⁷ M, at least 10⁻⁹ M, and at least 10⁻¹¹ M.

8. The fragment of any preceding claim, wherein said fragment is an Fab' fragment, an F(ab')₂ fragment, an Fab fragment, or an Fᵥ fragment of said monoclonal antibody.

9. A human monoclonal antibody or fragment thereof according to any of claims 1-8, for use in therapy.

10. A human monoclonal antibody or fragment thereof according to any of claims 1-8, for use in (i) preventing or treating an antibody-mediated disease in a patient, (ii) preventing or treating an IgE-mediated disease in a patient, or (iii) preventing or treating an autoimmune disease in a patient.

11. Use of a human monoclonal antibody or fragment thereof according to any of claims 1-8 in the manufacture of a medicament for (i) preventing or treating an antibody-mediated disease in a patient, (ii) preventing or treating an IgE-mediated disease in a patient, or (iii) preventing or treating an autoimmune disease in a patient.

12. A human monoclonal antibody or fragment thereof according to any of claims 1-8, for use in inhibiting antibody production by B cells in a human patient.

13. Use of a human monoclonal antibody or fragment thereof according to any of claims 1-8 in the manufacture of a medicament for inhibiting antibody production by B cells in a human patient.

14. A method for inhibiting growth or differentiation of a normal human B cell, or for inhibiting proliferation of a normal human B cell, wherein said proliferation is augmented by the interaction of a CD40 ligand with a CD40 antigen expressed on the surface of a B cell, said method comprising contacting said B cell *in vitro* with an effective amount of a human anti-CD40 monoclonal antibody or fragment thereof according to any of claims 1-8.

15. A hybridoma capable of producing a human monoclonal antibody according to any of claims 1 to 7.

16. A nucleic acid comprising a polynucleotide that encodes a human monoclonal antibody or fragment thereof according to any of claims I to 8.

17. The nucleic acid according to claim 16, which comprises the polynucleotide sequence set forth in SEQ ID NO:7 or SEQ ID NO:8.

18. A pharmaceutical composition comprising a human monoclonal antibody or fragment thereof according to any of claims 1-8 and a pharmaceutically acceptable excipient.

## Patentansprüche

1. Humaner monoklonaler Antikörper oder Fragment desselben, der geeignet ist spezifisch an das humane auf der Oberfläche einer normalen humanen B-Zelle exprimierte CD40-Antigen zu binden, wobei der monoklonale Antikörper oder das Fragment keine signifikante agonistische Aktivität aufweist, wenn der monoklonale Antikörper oder das Fragment an das auf der Oberfläche einer normalen B-Zelle exprimierte CD40-Antigen bindet, wodurch, wenn der monoklonale Antikörper oder das Fragment an das auf der Oberfläche der normalen B-Zelle exprimierte CD40-Antigen bindet, das Wachstum oder die Differenzierung der normalen B-Zelle verhindert wird, wobei der monoklonale Antikörper oder das Fragment desselben einen variablen Bereich der schweren Kette umfasst, der die Reste 31-35, 50-66 und 99-111 der SEQ ID Nr. 13 enthält, und einen variablen Bereich der leichten Kette umfasst, der die Reste 24-39, 55-61 und 94-102 der SEQ ID Nr. 17 enthält.

2. Humaner monoklonaler Antikörper oder Fragment desselben nach Anspruch 1, welcher die in SEQ ID Nr. 13 dargelegte Aminosäuresequenz umfasst.

3. Humaner monoklonaler Antikörper oder Fragment desselben nach Anspruch 1, welcher die in SEQ ID Nr. 17 dargelegte Aminosäuresequenz umfasst.

4. Humaner monoklonaler Antikörper oder Fragment desselben nach Anspruch 1, welcher die in SEQ ID Nr. 13 dargelegte Aminosäuresequenz und die in SEQ ID Nr. 17 dargelegte Aminosäuresequenz umfasst.

5. Humaner monoklonaler Antikörper oder Fragment desselben nach einem der vorhergehenden Ansprüche, welcher wenigstens eine der folgenden biologischen Aktivitäten besitzt:
(i) Inhibierung der Immunglobulinsekretion durch humane periphere durch T-Zellen stimulierte B-Zellen;
(ii) Inhibierung der Proliferation humaner peripherer durch Jurkat T-Zellen stimulierter B-Zellen; und
(iii) Inhibierung der Proliferation humaner peripherer durch CD40 exprimierende Zellen stimulierter B-Zellen.

6. Humaner monoklonaler Antikörper oder Fragment desselben nach einem der vorhergehenden Ansprüche, welcher die folgenden biologischen Aktivitäten besitzt:
(i) Inhibierung der Immunglobulinsekretion durch humane periphere durch T-Zellen stimulierte B-Zellen;
(ii) Inhibierung der Proliferation humaner peripherer durch Jurkat T-Zellen stimulierter B-Zellen; und
(iii) Inhibierung der Proliferation humaner peripherer durch CD40 exprimierende Zellen stimulierter B-Zellen.

7. Humaner monoklonaler Antikörper oder Fragment desselben nach einem der vorhergehenden Ansprüche, wobei der Antikörper oder das Fragment desselben an das humane CD40-Antigen mit einer Affinität (K_{D}), ausgewählt aus der Gruppe, bestehend aus wenigstens 10⁻⁵ M, wenigstens 10⁻⁷ M, wenigstens 10⁻⁹ M und wenigstens 10⁻¹¹ M, bindet.

8. Fragment nach einem der vorhergehenden Ansprüche, wobei das Fragment ein Fab'-Fragment, ein F(ab')₂-Fragment, einer Fab-Fragment oder ein Fᵥ-Fragment des monoklonalen Antikörpers ist.

9. Humaner monoklonaler Antikörper oder Fragment desselben nach einem der Ansprüche 1 bis 8 zur Verwendung in der Therapie.

10. Humaner monoklonaler Antikörper oder Fragment desselben nach einem der Ansprüche 1 bis 8 zur Verwendung (i) zur Vorbeugung oder Behandlung einer mit einem Antikörper in Zusammenhang stehenden Erkrankung in einem Patienten, (ii) zur Vorbeugung oder Behandlung einer mit IgE in Zusammenhang stehenden Erkrankung in einem Patienten oder (iii) zur Vorbeugung oder Behandlung einer Autoimmunerkrankung in einem Patienten.

11. Verwendung eines humanen monoklonalen Antikörpers oder Fragments desselben nach einem der Ansprüche 1 bis 8 in der Herstellung eines Arzneimittels (i) zur Vorbeugung oder Behandlung einer mit einem Antikörper in Zusammenhang stehenden Erkrankung in einem Patienten, (ii) zur Vorbeugung oder Behandlung einer mit IgE in Zusammenhang stehenden Erkrankung in einem Patienten oder (iii) zur Vorbeugung oder Behandlung einer Autoimmunerkrankung in einem Patienten

12. Humaner monoklonaler Antikörper oder Fragment desselben nach einem der Ansprüche 1 bis 8 zur Verwendung beim Inhibieren der Antikörperproduktion von B-Zellen in einem menschlichen Patient.

13. Verwendung eines humanen monoklonalen Antikörpers oder Fragments desselben nach einem der Ansprüche 1 bis 8 in der Herstellung eines Arzneimittels zum Inhibieren der Antikörperproduktion von B-Zellen in einem menschlichen Patient.

14. Verfahren zum Inhibieren des Wachstums oder der Differenzierung einer normalen humanen B-Zelle, oder zum Inhibieren der Proliferation einer normalen humanen B-Zelle, wobei die Proliferation durch die Wechselwirkung eines CD40-Liganden mit einem auf der Oberfläche einer B-Zelle exprimierten CD40-Antigens erhöht wird, wobei das Verfahren das in Kontakt bringen der B-Zelle *in vitro* mit einer wirksamen Menge des humanen monoklonalen anti-CD40-Antikörpers oder Fragments desselben gemäß einem der Ansprüche 1 bis 8 umfasst.

15. Hybridom, das geeignet ist einen humanen monoklonalen Antikörper gemäß einem der Ansprüche 1 bis 7 herzustellen.

16. Nukleinsäure, umfassend ein Polynukleotid, das für einen humanen monoklonalen Antikörper oder ein Fragment desselben gemäß einem der Ansprüche 1 bis 8 codiert.

17. Nukleinsäure nach Anspruch 16, welche die in SEQ ID Nr. 7 oder SEQ ID Nr. 8 dargelegte Polynukleotidsequenz umfasst.

18. Pharmazeutische Zusammensetzung, umfassend einen humanen monoklonalen Antikörper oder ein Fragment desselben gemäß einem der Ansprüche 1 bis 8 und einen pharmazeutisch verträglichen Arzneistoffträger.

## Revendications

1. Anticorps monoclonal humain ou fragment de celui-ci, capable de se lier spécifiquement à un antigène CD40 humain exprimé à la surface d'un lymphocyte B humain normal, ledit anticorps monoclonal ou fragment étant dépourvu d'activité agonistique significative lorsque ledit anticorps monoclonal ou fragment se lie à l'antigène CD40 exprimé à la surface dudit lymphocyte B normal, de sorte que, lorsque ledit anticorps monoclonal ou fragment se lie à l'antigène CD40 exprimé à la surface dudit lymphocyte B normal, la croissance ou différentiation dudit lymphocyte B normal est inhibée, ledit anticorps monoclonal ou fragment de celui-ci comprenant une région variable de chaîne lourde contenant les résidus 31 à 35, 50 à 66 et 99 à 111 de SEQ ID n° 13, et comprenant une région variable de chaîne légère contenant les résidus 24 à 39, 55 à 61 et 94 à 102 de SEQ ID n° 17.

2. Anticorps monoclonal humain ou fragment de celui-ci selon la revendication 1, lequel comprend la séquence d'acides aminés indiquée dans SEQ ID n° 13.

3. Anticorps monoclonal humain ou fragment de celui-ci selon la revendication 1, lequel comprend la séquence d'acides aminés indiquée dans SEQ ID n° 17.

4. Anticorps monoclonal humain ou fragment de celui-ci selon la revendication 1, lequel comprend la séquence d'acides aminés indiquée dans SEQ ID n° 13 et la séquence d'acides aminés indiquée dans SEQ ID n° 17.

5. Anticorps monoclonal humain ou fragment de celui-ci selon l'une quelconque des revendications précédentes, lequel possède au moins l'une des activités biologiques suivantes :
(i) inhibition de la sécrétion d'immunoglobulines par les lymphocytes B périphériques humains stimulés par les lymphocytes T ;
(ii) inhibition de la prolifération des lymphocytes B périphériques humains stimulée par les lymphocytes T Jurkat ; et
(iii) inhibition de la prolifération des lymphocytes B périphériques humains stimulée par les cellules exprimant CD40L.

6. Anticorps monoclonal humain ou fragment de celui-ci selon l'une quelconque des revendications précédentes, lequel possède les activités biologiques suivantes :
(i) inhibition de la sécrétion d'immunoglobulines par les lymphocytes B périphériques humains stimulés par les lymphocytes T ;
(ii) inhibition de la prolifération des lymphocytes B périphériques humains stimulée par les lymphocytes T Jurkat ; et
(iii) inhibition de la prolifération des lymphocytes B périphériques humains stimulée par les cellules exprimant CD40L.

7. Anticorps monoclonal humain ou fragment de celui-ci selon l'une quelconque des revendications précédentes, lequel anticorps ou fragment de celui-ci se lie audit antigène CD40 humain avec une affinité (K_{D}) choisie dans le groupe constitué d'au moins 10⁻⁵ M, au moins 10⁻⁷ M, au moins 10⁻⁹ M, et au moins 10⁻¹¹ M.

8. Fragment selon l'une quelconque des revendications précédentes, lequel fragment est un fragment Fab', un fragment F(ab')₂, un fragment Fab, ou un fragment Fᵥ dudit anticorps monoclonal.

9. Anticorps monoclonal humain ou fragment de celui-ci selon l'une quelconque des revendications 1 à 8, pour une utilisation en thérapie.

10. Anticorps monoclonal humain ou fragment de celui-ci selon l'une quelconque des revendications 1 à 8, pour une utilisation dans (i) la prévention ou le traitement d'une maladie médiée par anticorps chez un patient, (ii) la prévention ou le traitement d'une maladie médiée par IgE chez un patient, ou (iii) la prévention ou le traitement d'une maladie auto-immune chez un patient.

11. Utilisation d'un anticorps monoclonal humain ou d'un fragment de celui-ci selon l'une quelconque des revendications 1 à 8 dans la fabrication d'un médicament destiné à (i) prévenir ou traiter une maladie médiée par anticorps chez un patient, (ii) prévenir ou traiter une maladie médiée par IgE chez un patient, ou (iii) prévenir ou traiter une maladie auto-immune chez un patient.

12. Anticorps monoclonal humain ou fragment de celui-ci selon l'une quelconque des revendications 1 à 8, destiné à inhiber la production d'anticorps par les lymphocytes B chez un patient humain.

13. Utilisation d'un anticorps monoclonal humain ou d'un fragment de celui-ci selon l'une quelconque des revendications 1 à 8 dans la fabrication d'un médicament destiné à inhiber la production d'anticorps par les lymphocytes B chez un patient humain.

14. Procédé pour inhiber la croissance ou la différentiation d'un lymphocyte B humain normal, ou pour inhiber la prolifération d'un lymphocyte B humain normal, dans lequel ladite prolifération est augmentée par l'interaction d'un ligand du CD40 avec un antigène CD40 exprimé à la surface d'un lymphocyte B, ledit procédé comprenant la mise en contact dudit lymphocyte B *in vitro* avec une quantité efficace d'un anticorps monoclonal anti-CD40 humain ou d'un fragment de celui-ci selon l'une quelconque des revendications 1 à 8.

15. Hybridome capable de produire un anticorps monoclonal humain selon l'une quelconque des revendications 1 à 7.

16. Acide nucléique comprenant un polynucléotide qui code pour un anticorps monoclonal humain ou un fragment de celui-ci selon l'une quelconque des revendications 1 à 8.

17. Acide nucléique selon la revendication 16, lequel comprend la séquence polynucléotidique indiquée dans SEQ ID n° 7 ou SEQ ID n° 8.

18. Composition pharmaceutique comprenant un anticorps monoclonal humain ou un fragment de celui-ci selon l'une quelconque des revendications 1 à 8 et un excipient pharmaceutiquement acceptable.
